# EUROPEAN PATENT SPECIFICATION

(11) **EP 1 575 922 B1**
(45) Date of publication and mention of the grant of the patent: **14.05.2008**
(21) Application number: 03813891.3
(22) Date of filing: 15.12.2003
(51) Int. Cl.: C07D 231/14, C07D 231/16, C07D 207/34, C07D 277/56, C07D 263/34, C07D 333/38, C07D 307/68, C07D 309/28, C07D 327/06, C07D 213/82, C07D 239/28, C07D 239/30, C07D 237/24, C07C 233/03, C07C 211/45

(54) **BIPHENYL DERIVATIVES AND THEIR USE AS FUNGICIDES**
BIPHENYLDERIVATE UND DEREN VERWENDUNG ALS FUNGIZIDE
DERIVES DE BIPHENYLE ET LEUR UTILISATION EN TANT QUE FONGICIDES

(30) Priority: 24.12.2002 GB 0230155
(43) Date of publication of application: 21.09.2005
(73) Proprietor: Syngenta Participations AG, 4058 Basel (CH)
(72) Inventor: EHRENFREUND, Josef, CH-4058 Basel (CH); LAMBERTH, Clemens, CH-4058 Basel (CH); TOBLER, Hans, CH-4058 Basel (CH); WALTER, Harald, CH-4058 Basel (CH)
(74) Representative: Hölscher, Ingo
(86) International application number: PCT/EP2003/014248
(87) International publication number: WO 2004/058723

(56) References cited:
- WO-A-02/08197
- DE-A- 2 205 732
- DE-A- 10 204 390
- DE-A- 10 204 391
- DE-A- 10 215 292
- DE-A- 10 218 231
- JP-A- 2001 302 605
- US-A- 3 928 364
- US-A- 4 016 214
- US-A- 4 036 989
- D. C. NAAE ET. AL. : "The solid-gas chlorination and bromination of a fluorinated olefin." TETRAHEDRON LETTERS, vol. 32, 1976, pages 2761-4, XP001181810
- J. L. CHANAL ET. AL. : "Comparison of the Metabolism and Pharmacokinetics of Metbufen and Itanoxone and their Analogues in Rats." ARZNEIMITTEL FORSCHUNG, vol. 38, no. 10, 1988, pages 1454-60, XP000674615
- D. G. NAAE: "Reaction of Crystalline Fluoro Olefins with Bromine Vapour." JOURNAL OF ORGANIC CHEMISTRY, vol. 42, no. 10, 1977, pages 1780-3, XP002230049
- M. W. RENOLL: "Vinyl Aromatic Compounds. III. Fluorinated Derivatives. " JOURNAL OF THE AMERICAN CHEMICAL SOCIETY, vol. 68, 1946, pages 1159-61, XP001181808
- H. COUSSE ET. AL. : "Synthèse, structure et activité hypocholerérolémiante d'une série d'acides gamma-aryl, gamma-oxo butyriques substitués et dérivés. " EUROPEAN JOURNAL OF MEDICINAL CHEMISTRY , vol. 22, 1987, pages 45-57, XP001181809

## Description

The present invention relates to novel carboxamide derivatives as active ingredients which have microbiocidal activity, in particular fungicidal activity. The invention also relates to preparation of these active ingredients, to novel diphenyl derivatives used as intermediates in the preparation of these active ingredients, to preparation of these novel intermediates, to agrochemical compositions which comprise at least one of the novel active ingredients, to preparation of these compositions and to use of the active ingredients or compositions in agriculture or horticulture for controlling or preventing infestation of plants by phytopathogenic microorganisms, preferably fungi.

Fungicidally active carboxamide derivatives are disclosed in JP2001072510, JP2001072508; JP2001072507 and JP2001302605.

Certain amino- or halo-substituted diphenyl derivatives are disclosed in DE2205732. WO 03/91240 discloses fungicidally active thienyl-carboxamides. JP-A-2001-302605 discloses fungicidally active biphenyl derivatives of formula IA 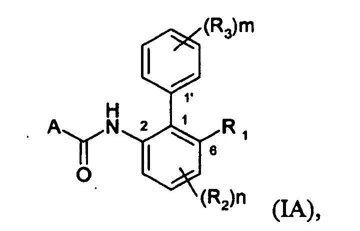 wherein A can be pyrazole or thiazole group and the optional substituent R₃ can be alkinyl. R₁ is selected from chlorine and optionally linked/substituted alkyl, alkenyl, alkinyl, cycloalkyl or cycloalkenyl.

WO 03/70705 and WO 03/66609 disclose fungicidally active biphenyl derivatives of formula IB 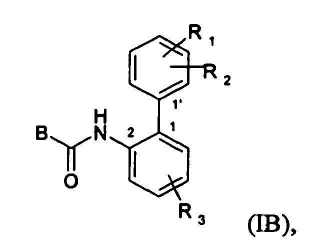 wherein B is pyrazole or thiazole group respectively and the substitutents R₁ and R₂ are selected from halogen, cyano, nitro and optionally linked/substitued alkyl, alkenyl or cycloalkyl. R₃ is fluorine in WO 03/70705 and absent in WO 03/66609.

WO 03/66610 discloses fungicidally active biphenyl derivatives of formula IC 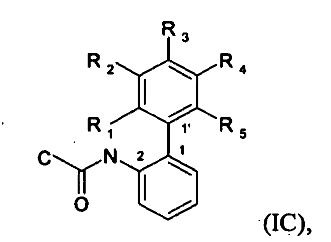 wherein C is a thiazole group and the substitutents R₁ to R₅ are selected from hydrogen, halogen, cyano, nitro and optionally linked/substitued alkyl, alkenyl or cycloalkyl and at least one of R₁-R₅ must be present.

The present invention provides a compound of formula (I): 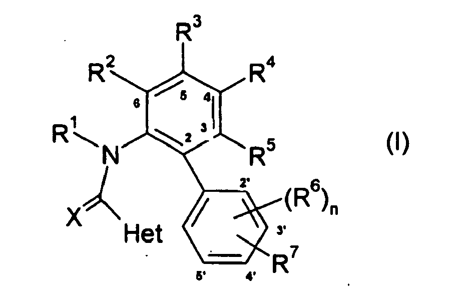 where Het is a pyrazole, pyrrole or thiazole ring, the ring being substituted by one, two or three groups R^{y};
R¹ is hydrogen;
R², R³ and R⁴ are each, independently, hydrogen, halogen, methyl or CF₃;
R⁵ is hydrogen or fluorine;
each R⁶ is, independently, halogen, methyl or CF₃;
R⁷ is (Z)ₘC≡C(Y¹);
each R^{y} is, independently, halogen, C₁₋₃ alkyl, C₁₋₃ haloalkyl, C₁₋₃ alkoxy(C₁₋₃)alkylene or cyano;
X is O;
Y¹ is hydrogen, halogen, C₁₋₄ alkyl [optionally substituted by one or more substituents each independently selected from halogen, hydroxy, C₁₋₄ alkoxy, C₁₋₄ haloalkoxy, C₁₋₄ alkylthio, C₁₋₄ haloalkylthio, C₁₋₄ alkylamino, di(C₁₋₄)alkylamino, C₁₋₄ alkoxycarbonyl and tri(C₁₋₄)alkylsilyl], C₂₋₄ alkenyl [optionally substituted by one or more substituents each independently selected from halogen], C₂₋₄ alkynyl [optionally substituted by one or more substituents each independently selected from halogen], C₃₋₇ cycloalkyl [optionally substituted by one or more substituents each independently selected from halogen, C₁₋₄ alkyl and C₁₋₄ haloalkyl] or tri(C₁₋₄)alkylsilyl; Z is C₁₋₄ alkylene [optionally substituted by one or more substituents each independently selected from hydroxy, cyano, C₁₋₄ alkoxy, C₁₋₄ haloalkoxy, C₁₋₄ alkylthio, COOH and
COO-C₁₋₄ alkyl];
m is 0 or 1; and
n is 0, 1 or 2.

In one aspect, the present invention provides a compound of formula (I) as defined above; one of R², R³, R⁴ and R⁵ is fluorine and the others are all hydrogen; n is 1; and Het is

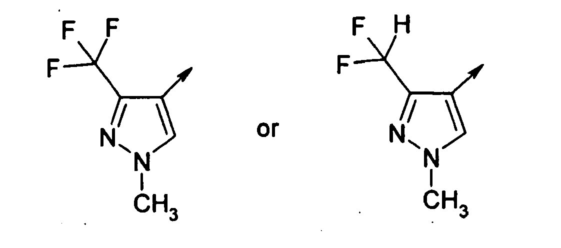

In another aspect, the present invention provides a compound of formula (I) as defined above; R¹, R², R³, R⁴ and R⁵ are all hydrogen; and Het is

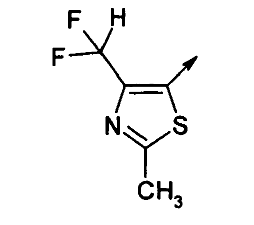

In a further aspect, the present invention provides a compound of formula (I) as defined above; R¹, R², R³, R⁴ and R⁵ are all hydrogen; n is 1; and Het is

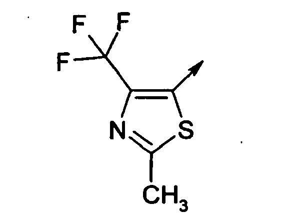

Each alkyl moiety is a straight or branched chain and is, for example, methyl, ethyl, n-propyl, n-butyl, iso-propyl, n-butyl, sec-butyl, iso-butyl or tert-butyl. Likewise, each alkylene moiety is a straight or branched chain.

Haloalkyl moieties are alkyl moieties which are substituted by one or more of the same or different halogen atoms and are, for example, CF₃, CF₂Cl, CHF₂, CH₂F, CCl₃, CF₃CH₂, CHF₂CH₂, CH₂FCH₂, CH₃CHF or CH₃CF₂.

Alkenyl and alkynyl moieties can be in the form of straight or branched chains. The alkenyl moieties, where appropriate, can be of either the (E)- or (Z)-configuration. Examples are vinyl, allyl, ethynyl and propargyl.

Cycloalkyl includes cyclopropyl, cyclobutyl, cyclopentyl, cyclohexyl and cycloheptyl.

In tri(C₁₋₄)alkylsilyl and in di(C₁₋₄)alkylamino, each alkyl moiety is selected independently.

Throughout this description, Me stands for methyl and Et stands for ethyl.

Preferably R² is hydrogen.

Preferably R³ is hydrogen.

Preferably R⁴ is hydrogen.

Preferably R⁵ is hydrogen or halogen.

More preferably R⁵ is hydrogen or fluorine.

Even more preferably R⁵ is hydrogen.

Preferably R⁷ is in the 4' position.

Preferably R⁷ is propargyl [optionally substituted by one to three substituents each independently selected from halogen, C₁₋₄ alkyl, C₁₋₂ haloalkyl and trimethylsilyl].

In one particular aspect R⁷ is propargyl [optionally substituted by one to three substituents each independently selected from halogen, CH₃, C₁₋₂ haloalkyl and trimethylsilyl].

More preferably R⁷ is C≡CH, C≡CSiMe₃, C≡CSiEt₃, C≡CSiMe₂C(CH₃)₃, C≡CCl, C≡CBr, C≡CF, C≡CCF₃, C≡CCF₂H, C≡CCF₂Cl, C≡CCF₂Me, C≡CCF₂Et, C≡CCHFCl, C≡CCF₂Br, C≡CC₂F₅, C≡CCF(CF₃)₂, C≡CCHF(CF₃), C≡CCH₂F, C≡CCH(Me)F, C≡CCH(Et)F, C≡CMe, C≡CCH₂Me, C≡CCHMe₂, C≡CCH₂CHMe₂, C≡CCMe₃, C≡CCH₂CMe₃, C≡CCH₂SiMe₃, C≡CCMe₂Cl, C≡CCMe₂F, C≡CCH₂OMe, C≡CCH₂CF₃, C≡CCMe₂OMe, C≡CCMe₂OH, C≡CC(Me)=CH₂, C≡CCF=CF₂, C≡C(cyclopropyl), C≡C(cyclopentyl), C≡C(1-F-cyclopentyl), CH₂C≡CH, CF₂C≡CH, CHFC≡CH, CH(CF₃)C≡CH, CH₂C≡CCMe₃ or CH₂C≡CSiMe₃.

In one particular aspect R⁷ is more preferably C≡CH, C≡CSiMe₃, C≡CSiEt₃, C≡CSiMe₂C(CH₃)₃, C≡CCl, C≡CBr, C≡CCF₃, C≡CCF₂H, C≡CCF₂Cl, C≡CCF₂Br, C≡CCF(CF₃)₂C≡CMe, C≡CCHMe₂, C≡CCMe₃, C≡CCMe₂Cl, C≡CCH₂OMe, C≡C(cycloC₃H₅), C≡C(cycloC₅H₉), CH₂C≡CH or CH₂C≡CSiMe₃.

Even more preferably k⁷ is C≡CH, C≡CSiMe₃, C≡CSiEt₃, C≡CSiMe₂C(CH₃)₃, C≡CCl, C≡CCF₃, C≡CCF₂H, C≡CCF₂Cl, C≡CCHFCl, C≡CCF₂Me, C≡CCF₂Et, C≡CCHFEt, C≡CCF₂Br, C≡CCF(CF₃)₂, C≡CCF₂CF₃, C≡CCHF(CF₃), C≡CCH₂F, C≡CCH(Me)F, C≡CMe, C≡CCHMe₂, C≡CCH₂Me, C≡CCH₂CHMe₂, C≡CCMe₃, C≡CCH₂CMe₃, C≡CCMe₂F, C≡CCH₂CF₃, C≡C(cyclopropyl), C≡C(cyclopentyl), C≡C(1-F-cyclopentyl), C≡CC(Me)=CH₂, C≡CCF=CF₂, C≡CCH₂SiMe₃, CH₂C≡CH, CF₂C≡CH or CHFC≡CH.

In one particular aspect R⁷ is even more preferably C≡CH, C≡CSiMe₃, C≡CCl, C≡CBr, C≡CCF₃, C≡CMe, C≡CCMe₃, C≡CCHMe₂, C≡C(cycloC₃H₅), CH₂C≡CH, SiMe₃ or CH₂C≡CSiMe₃.

Yet more preferably R⁷ is C≡CH, C≡CSiMe₃, C≡CCl, C≡CCF₃, C≡CCF₂H, C≡CCHFCl, C≡CCHF(CF₃), C≡CCF₂Cl, C≡CCF₂Me, C≡CCF₂Br, C≡CCF₂CF₃, C≡CCH₂F, C≡CCH(Me)F, C≡CMe, C≡CCHMe₂, C≡CCH₂CHMe₂, C≡CCMe₃, C≡CCCH₂CMe₃, C≡CCMe₂F, C≡CCH₂CF₃, C≡C(cyclopropyl), C≡C(cyclopentyl), C≡C(1-F-cyclopentyl), CH₂C≡CH, CF₂C≡CH, CHFC≡CH or C≡CCH₂Me.

In one particular aspect R⁷ is yet more preferably C≡CH, C≡CSiMe₃, C≡CCl, C≡CBr, C≡CCF₃, C≡CMe, C≡CCMe₃, C≡CCHMe₂, C≡C(cycloC₃H₅), CH₂C≡CH or CH₂C≡CSiMe₃.

Preferably nitrogen atoms in the Het ring are, independently, either unsubstituted or substituted by R^{y}.

When R^{y} is a substituent on a nitrogen atom it is preferably C₁₋₃ alkyl, C₁₋₃ haloalkyl or methoxymethylene; more preferably C₁₋₂ alkyl, CF₃, CF₂Cl, CHF₂, CH₂F or methoxymethylene; even more preferably methyl, CHF₂ or methoxymethylene; yet more preferably methyl or methoxymethylene; and most preferably methyl.

Preferably carbon atoms in the Het ring which are not bonded to the atom substituted by CXNR¹ are, independently, either unsubstituted or substituted by R^{y}.

When R^{y} is a substituent on a carbon atom which is not bonded to the atom substituted by CXNR¹ it is preferably halogen, C₁₋₃ alkyl, C₁₋₃ haloalkyl or methoxymethylene; more preferably chloro, methoxymethylene, CH₃, CHF₂ or CF₃; yet more preferably chloro, CH₃, CHF₂ or CF₃; and even more preferably CH₃ or CF₃.There may be one or two carbon atoms in the Het ring bonded to the atom substituted by CXNR¹; preferably such carbon atoms are, independently, either unsubstituted or substituted by R^{y}.

When R^{y} is a substituent on a carbon atom bonded to the atom substituted by CXNR¹ it is preferably halogen, C₁₋₃ alkyl or C₁₋₃ haloalkyl; more preferably chloro, fluoro, bromo, C₁₋₂ alkyl, CF₃, CF₂Cl, CHF₂, CH₂F; and even more preferably chloro, fluoro, bromo, methyl, CF₃, CHF₂ or CH₂F.

More preferably, when there is only one carbon atom in the Het ring bonded to the atom substituted by CXNR¹ that carbon atom is substituted by R^{y}.

More preferably, when there are two carbon atoms in the Het ring bonded to the atom substituted by CXNR¹ one such carbon atom is substituted by R^{y} and the other carbon atom is either unsubstituted or is substituted by fluoro, chloro or methyl.

Preferably m is 0.

Preferably n is 0.

Preferably X is O.

Compounds of formula (II): 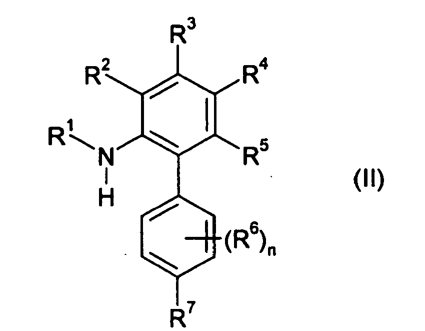 where R¹, R², R³, R⁴, R⁵, R⁶, R⁷ and n are as defined above for a compound of formula (I), are also novel and are useful as intermediates in the preparation of compounds of formula (I).

Therefore, in another aspect the present invention provides a compound of formula (II), where R¹, R², R³, R⁴, R⁵, R⁶, R⁷ and n are as defined above for a compound of formula (I).

The preferred values for R¹, R², R³, R⁴, R⁵, R⁶, R⁷ and n for a compound of formula (II) are as defined above for a compound of formula (I).

Many compounds of formula (III): 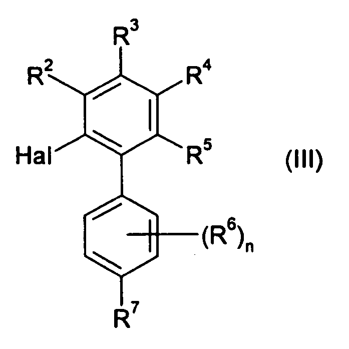 where R², R³, R⁴, R⁵, R⁶, R⁷ and n are as defined above for a compound of formula (I) and Hal is bromo, chloro or iodo, are also novel and are useful as intermediates in the preparation of compounds of formula (I).

Certain compounds of formula (III) are already known; Table 0 shows known compounds of formula (IIIa) where Hal, R², R³, R⁴ and R⁷ are as defined in Table 0.

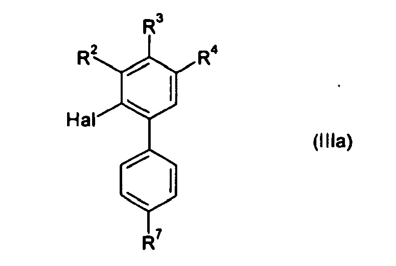

**Table 0**

| **Hal** | **R²** | **R³** | **R⁴** | **R⁷** |
|---|---|---|---|---|
| Cl | Cl | H | H | C(CH₃)=CH-CH₂-OH |
| Br | H | Me | H | C(CF₃)=CF₂ |
| Br | H | Me | Br | C(CF₃)=CF₂ |
| Cl | H | H | H | C≡CH |
| Cl | H | H | H | CH=CH-CH₂-CH₂-OH |
| Cl | H | H | H | C(CH₃)=CH-CH₂-OH |
| Cl | H | H | H | C(CH₃)=CH-C(=O)-OC₂H₅ |
| Cl | H | H | H | C(CH₃)=CH-CH(OH)CH₃ |
| Cl | H | H | H | CH=CH-CH(OH)CH₃ |

Therefore, in a further aspect the present invention provides a compound of formula (III), where R², R³, R⁴, R⁵, R⁶, R⁷ and n are as defined above for a compound of formula (I) and Hal is bromo, chloro or iodo; provided that the compound is not a compound of formula (IIIa) according to Table 0, wherein Hal is chloro, R₂, R₃ and R₄ are hydrogen and R₇ is C≡CH.

The preferred values for R², R³, R⁴, R⁵, R⁶, R⁷ and n for a compound of formula (III) are as defined above for a compound of formula (I).

Preferably Hal is bromo or chloro.

More preferably Hal is bromo.

The compounds of formulae (I), (II) and (III) may exist as different geometric or optical isomers or in different tautomeric forms. For each formula, this invention covers all such isomers and tautomers and mixtures thereof in all proportions as well as isotopic forms such as deuterated compounds.

The compounds in Tables 1 to 5 below illustrate compounds of the invention.

**Table 1**

| **Compound No.** | **R¹** | **R⁷** | **R⁸** | **R⁹** | **R¹⁰** | **X** |
|---|---|---|---|---|---|---|
| 1.01 | H | C≡CH | H | Me | CF₃ | O |
| 1.02 | H | C≡CH | H | Me | CF₂H | O |
| 1.03 | H | C≡CH | F | Me | Me | O |
| 1.04 | H | C≡CH | H | CH₂OMe | CF₃ | O |
| 1.05 | H | C≡CSiMe₃ | H | Me | CF₃ | O |
| 1.06 | H | C≡CSiMe₃ | H | Me | CF₂H | O |
| 1.07 | H | C≡CSiMe₃ | F | Me | Me | O |
| 1.08 | H | C≡CCl | H | Me | CF₃ | O |
| 1.09 | H | C≡CCl | H | Me | CF₂H | O |
| 1.10 | H | C≡CCl | F | Me | Me | O |
| 1.11 | H | C≡CBr | H | Me | CF₃ | O |
| 1.12 | H | C≡CBr | H | Me | CF₂H | O |
| 1.13 | H | C≡CBr | F | Me | Me | O |
| 1.14 | H | C≡CCF₃ | H | Me | CF₃ | O |
| 1.15 | H | C≡CCF₃ | H | Me | CF₂H | O |
| 1.16 | H | C≡CCF₃ | F | Me | Me | O |
| 1.17 | H | CH₂C≡CH | H | Me | CF₃ | O |
| 1.18 | H | CH₂C≡CH | H | Me | CF₂H | O |
| 1.19 | H | CH₂C≡CH | F | Me | Me | O |
| 1.20 | H | CH₂C≡CH | H | CH₂OMe | CF₃ | O |
| 1.21 | H | CH₂C≡CSiMe₃ | H | Me | CF₃ | O |
| 1.22 | H | CH₂C≡CSiMe₃ | H | Me | CF₂H | O |
| 1.23 | H | CH₂C≡CSiMe₃ | F | Me | Me | O |
| 1.24 | H | C≡CCMe₃ | H | Me | CF₃ | O |
| 1.25 | H | C≡CCMe₃ | H | Me | CF₂H | O |
| 1.26 | H | C≡CCMe₃ | F | Me | Me | O |
| 1.27 | H | C≡CMe | H | Me | CF₃ | O |
| 1.28 | H | C≡CMe | H | Me | CF₂H | O |
| 1.29 | H | C≡CMe | F | Me | Me | O |
| 1.30 | H | C≡CH | H | CF₂H | CF₂H | O |
| 1.31 | H | C≡CH | H | CF₂H | CF₃ | O |
| 1.32 | H | C≡CH | H | Me | CH₂F | O |
| 1.33 | H | C≡CSiMe₃ | H | Me | CH₂F | O |
| 1.34 | H | C≡C(cyclopropyl) | H | Me | CF₃ | O |
| 1.35 | H | C≡C(cyclopropyl) | H | Me | CHF₂ | O |
| 1.36 | H | C≡CF | H | Me | CF₃ | O |
| 1.37 | H | C≡CF | H | Me | CF₂H | O |
| 1.38 | H | C≡CF | F | Me | Me | O |
| 1.39 | H | C≡CCF₂Cl | H | Me | CF₃ | O |
| 1.40 | H | C≡CCF₂Cl | H | Me | CF₂H | O |
| 1.41 | H | C≡CCF₂Cl | F | Me | Me | O |
| 1.42 | H | C≡CCF₂H | H | Me | CF₃ | O |
| 1.43 | H | C≡CCF₂H | H | Me | CF₂H | O |
| 1.44 | H | C≡CCF₂H | F | Me | Me | O |
| 1.45 | H | C≡CCF₂Br | H | Me | CF₃ | O |
| 1.46 | H | C≡CCF₂Br | H | Me | CF₂H | O |
| 1.47 | H | C≡CCF₂Br | F | Me | Me | O |
| 1.48 | H | C≡CCH₂F | H | Me | CF₃ | O |
| 1.49 | H | C≡CCH₂F | H | Me | CF₂H | O |
| 1.50 | H | C≡CCH₂F | F | Me | Me | O |
| 1.51 | H | C≡CCH(Me)F | H | Me | CF₃ | O |
| 1.52 | H | C≡CCH(Me)F | H | Me | CF₂H | O |
| 1.53 | H | C≡CCH(Me)F | F | Me | Me | O |
| 1.54 | H | C≡CC(Me)₂F | H | Me | CF₃ | O |
| 1.55 | H | C≡CC(Me)₂F | H | Me | CF₂H | O |
| 1.56 | H | C≡CC(Me)₂F | F | Me | Me | O |
| 1.57 | H | C≡CCH₂C(Me)₃ | H | Me | CF₃ | O |
| 1.58 | H | C≡CCH₂C(Me)₃ | H | Me | CF₂H | O |
| 1.59 | H | C≡CCH₂C(Me)₃ | F | Me | Me | O |
| 1.60 | H | C≡CCH(Me)₂ | H | Me | CF₃ | O |
| 1.61 | H | C≡CCH(Me)₂ | H | Me | CF₂H | O |
| 1.62 | H | C≡CCH(Me)₂ | F | Me | Me | O |
| 1.63 | H | C≡CCH₂CH(Me)₂ | H | Me | CF₃ | O |
| 1.64 | H | C≡CCH₂CH(Me)₂ | H | Me | CF₂H | O |
| 1.65 | H | C≡CCH₂CH(Me)₂ | F | Me | Me | O |
| 1.66 | H | CH₂C≡CCMe₃ | H | Me | CF₃ | O |
| 1.67 | H | CH₂C≡CCMe₃ | H | Me | CF₂H | O |
| 1.68 | H | CH₂C≡CCMe₃ | F | Me | Me | O |
| 1.69 | H | CF₂C≡CCMe₃ | H | Me | CF₃ | O |
| 1.70 | H | CF₂C≡CCMe₃ | H | Me | CF₂H | O |
| 1.71 | H | CF₂C≡CCMe₃ | F | Me | Me | O |
| 1.72 | H | CF₂C≡CMe | H | Me | CF₃ | O |
| 1.73 | H | CF₂C≡CMe | H | Me | CF₂H | O |
| 1.74 | H | CF₂C≡CMe | F | Me | Me | O |
| 1.75 | H | CF₂C≡CH | H | Me | CF₃ | O |
| 1.76 | H | CF₂C≡CH | H | Me | CF₂H | O |
| 1.77 | H | CF₂C≡CH | F | Me | Me | O |
| 1.78 | H | CMe₂C≡CH | H | Me | CF₃ | O |
| 1.79 | H | CMe₂C≡CH | H | Me | CF₂H | O |
| 1.80 | H | CMe₂C≡CH | F | Me | Me | O |
| 1.81 | H | CHFC≡CH | H | Me | CF₃ | O |
| 1.82 | H | CHFC≡CH | H | Me | CF₂H | O |
| 1.83 | H | CHFC≡CH | F | Me | Me | O |
| 1.84 | H | CHMeC≡CH | H | Me | CF₃ | O |
| 1.85 | H | CHMeC≡CH | H | Me | CF₂H | O |
| 1.86 | H | CHMeC≡CH | F | Me | Me | O |
| 1.87 | H | CH(CF₃)C≡CH | H | Me | CF₃ | O |
| 1.88 | H | CH(CF₃)C≡CH | H | Me | CF₂H | O |
| 1.89 | H | CH(CF₃)C≡CH | F | Me | Me | O |
| 1.90 | H | C≡C (1-F-cyclopentyl) | H | Me | CF₃ | O |
| 1.91 | H | C≡C (1-F-cyclopentyl) | H | Me | CHF₂ | O |
| 1.92 | H | C≡CCH₂OMe | H | Me | CF₃ | O |
| 1.93 | H | C≡CCH₂OMe | H | Me | CF₂H | O |
| 1.94 | H | C≡CCH₂OMe | F | Me | Me | O |
| 1.95 | H | C≡CCMe₂OMe | H | Me | CF₃ | O |
| 1.96 | H | C≡CCMe₂OMe | H | Me | CF₂H | O |
| 1.97 | H | C≡CCMe₂OMe | F | Me | Me | O |
| 1.98 | H | C≡CCF₂Me | H | Me | CF₃ | O |
| 1.99 | H | C≡CCF₂Me | H | Me | CF₂H | O |
| 1.100 | H | C≡CCF₂Me | F | Me | Me | O |
| 1.101 | H | C≡CC(Me)=CH₂ | H | Me | CF₃ | O |
| 1.102 | H | C≡CC(Me)=CH₂ | H | Me | CF₂H | O |
| 1.103 | H | C≡ CCMe₂OH | H | Me | CF₃ | O |
| 1.104 | H | C≡ CCMe₂OH | H | Me | CF₂H | O |
| 1.105 | H | C≡ CSi(Me₂)CMe₃ | H | Me | CF₃ | O |
| 1.106 | H | C≡ CSi(Me₂)CMe₃ | H | Me | CF₂H | O |
| 1.107 | H | C≡ CCH₂SiMe₃ | H | Me | CF₃ | O |
| 1.108 | H | C≡ CCH₂SiMe₃ | H | Me | CF₂H | O |
| 1.109 | H | C≡ CCH₂SiMe₃ | F | Me | Me | O |
| 1.110 | H | C≡ CCMe₃ | H | CF₂H | CF₃ | O |
| 1.111 | H | C≡ CCH₂CF₃ | H | Me | CF₃ | O |
| 1.112 | H | C≡ CCH₂CF₃ | H | Me | CF₂H | O |
| 1.113 | H | C≡ CCH₂CF₃ | F | Me | Me | O |
| 1.114 | H | C≡ CCMe₃ | H | CF₂H | CF₂H | O |
| 1.115 | H | C≡ CCH₂CH₃ | H | Me | CF₃ | O |
| 1.116 | H | C≡ CCH₂CH₃ | H | Me | CF₂H | O |
| 1.117 | H | C≡ CCH₂CH₃ | F | Me | Me | O |
| 1.118 | H | C≡ CCF=CF₂ | H | Me | CF₃ | O |
| 1.119 | H | C≡ CCF=CF₂ | H | Me | CF₂H | O |
| 1.120 | H | C≡ CCHFCl | H | Me | CF₃ | O |
| 1.121 | H | C≡ CCHFCl | H | Me | CF₂H | O |
| 1.122 | H | C≡ CCHFCl | F | Me | Me | O |
| 1.123 | H | C≡ CCF₂CH₂CH₃ | H | Me | CF₃ | O |
| 1.124 | H | C≡ CCF₂CH₂CH₃ | H | Me | CF₂H | O |
| 1.125 | H | C≡ CCF₂CH₂CH₃ | F | Me | Me | O |
| 1.126 | H | C≡ CCHFCH₂CH₃ | H | Me | CF₃ | O |
| 1.127 | H | C≡ CCHFCH₂CH₃ | H | Me | CF₂H | O |
| 1.128 | H | C≡ CCHFCH₂CH₃ | F | Me | Me | O |
| 1.129 | H | C≡ CCF(CF₃)₂ | H | Me | CF₃ | O |
| 1.130 | H | C≡ CCF(CF₃)₂ | H | Me | CF₂H | O |
| 1.131 | H | C≡ CCF(CF₃)₂ | F | Me | Me | O |
| 1.132 | H | C≡ CC₂F₅ | H | Me | CF₃ | O |
| 1.133 | H | C≡ CC₂F₅ | H | Me | CF₂H | O |
| 1.134 | H | C≡ CC₂F₅ | F | Me | Me | O |

Table 1 provides 134 compounds of formula (Ia): 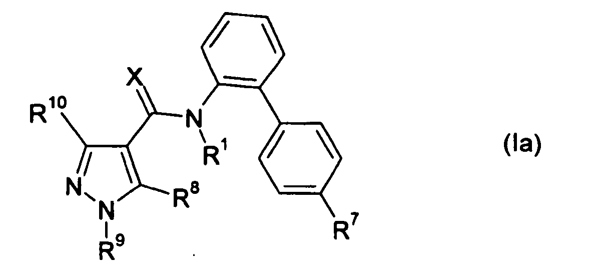 wherein R¹, R⁷, R⁸, R⁹, R¹⁰ and X are as defined in Table 1.

Table 1 provides 134 compounds of formula (IaA): 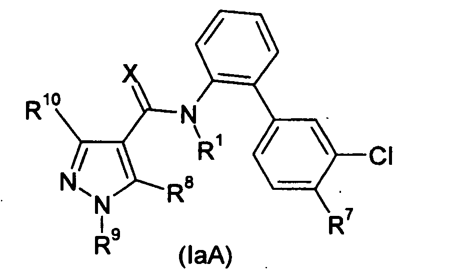 wherein R¹, R⁷, R⁸, R⁹, R¹⁰ and X are as defined in Table 1.

Table 1 provides 134 compounds of formula (IaB) wherein R¹, R⁷, R⁸, R⁹, R¹⁰ and X are as defined in Table 1.

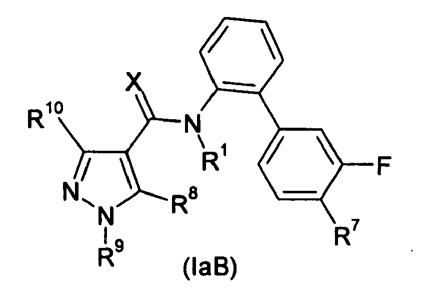

Table 1 provides 134 compounds of formula (IaC) wherein R¹, R⁷, R⁸, R⁹, R¹⁰ and X are as defined in Table 1.

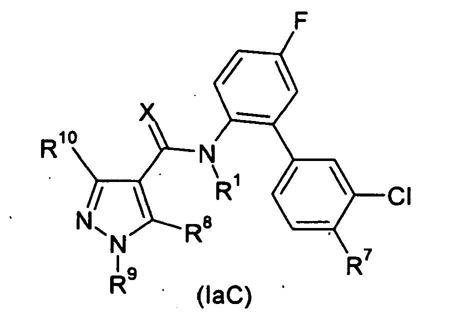

Table 1 provides 134 compounds of formula (IaD) wherein R¹, R⁷, R⁸, R⁹, R¹⁰ and X are as defined in Table 1.

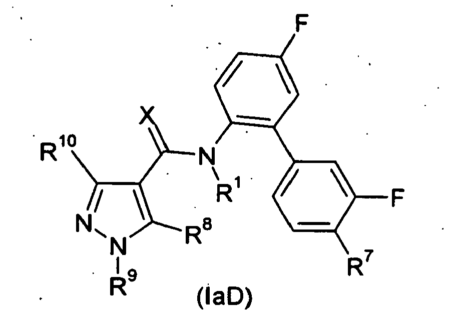

Table 1 provides 134 compounds of formula (IaE) wherein R¹, R⁷, R⁸, R⁹, R¹⁰ and X are as defined in Table 1.

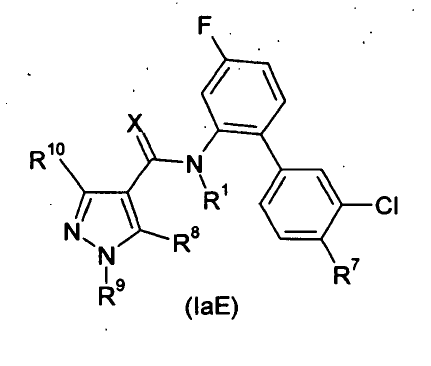

Table 1 provides 134 compounds of formula (IaF) wherein R¹, R⁷, R⁸, R⁹, R¹⁰ and X are as defined in Table 1.

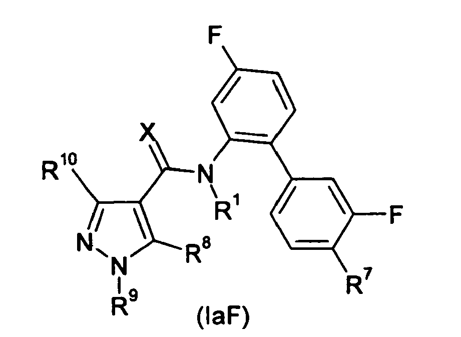

Table 1 provides 134 compounds of formula (IaG) wherein R¹, R⁷, R⁸, R⁹, R¹⁰ and X are as defined in Table 1.

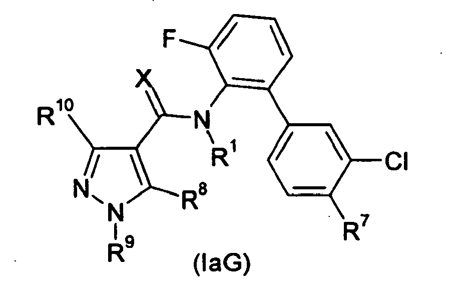

Table 1 provides 134 compounds of formula (IaH) wherein R¹, R⁷, R⁸, R⁹, R¹⁰ and X are as defined in Table 1.

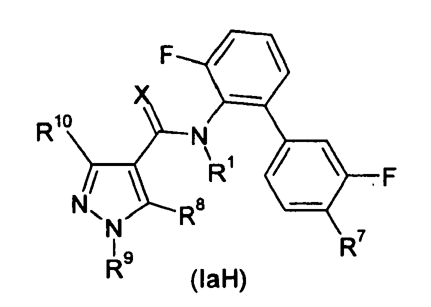

Table 1 provides 134 compounds of formula (IaI) wherein R¹, R⁷, R⁸, R⁹, R¹⁰ and X are as defined in Table 1.

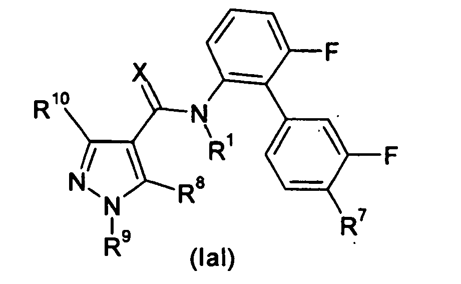

Table 1 provides 134 compounds of formula (IaJ) wherein R¹, R⁷, R⁸, R⁹, R¹⁰ and X are as defined in Table 1.

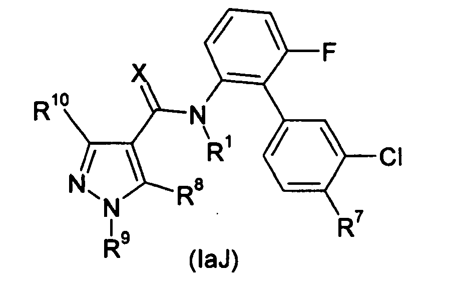

**Table 2**

| Compound No. | R¹ | R⁷ | R⁸ | R⁹ | R¹⁰ | X |
|---|---|---|---|---|---|---|
| 2.01 | H | C≡CH | H | Me | CF₃ | O |
| 2.02 | H | C≡CH | H | Me | CF₂H | O |
| 2.03 | H | C≡CH | F | Me | Me | O |
| 2.04 | H | C≡CH | H | CH₂OMe | CF₃ | O |
| 2.05 | H | C≡CSiMe₃ | H | Me | CF₃ | O |
| 2.06 | H | C≡CSiMe₃ | H | Me | CF₂H | O |
| 2.07 | H | C≡CSiMe₃ | F | Me | Me | O |
| 2.08 | H | C≡CCl | H | Me | CF₃ | O |
| 2.09 | H | C≡CCl | H | Me | CF₂H | O |
| 2.10 | H | C≡CCl | F | Me | Me | O |
| 2.11 | H | C≡CBr | H | Me | CF₃ | O |
| 2.12 | H | C≡CBr | H | Me | CF₂H | O |
| 2.13 | H | C≡CBr | F | Me | Me | O |
| 2.14 | H | C≡CCF₃ | H | Me | CF₃ | O |
| 2.15 | H | C≡CCF₃ | H | Me | CF₂H | O |
| 2.16 | H | C≡CCF₃ | F | Me | Me | O |
| 2.17 | H | CH₂C≡CH | H | Me | CF₃ | O |
| 2.18 | H | CH₂C≡CH | H | Me | CF₂H | O |
| 2.19 | H | CH₂C≡CH | F | Me | Me | O |
| 2.20 | H | CH₂C≡CH | H | CH₂OMe | CF₃ | O |
| 2.21 | H | CH₂C≡CSiMe₃ | H | Me | CF₃ | O |
| 2.22 | H | CH₂C≡CSiMe₃ | H | Me | CF₂H | O |
| 2.23 | H | CH₂C≡CSiMe₃ | F | Me | Me | O |
| 2.24 | H | C≡CCMe₃ | H | Me | CF₃ | O |
| 2.25 | H | C≡CCMe₃ | H | Me | CF₂H | O |
| 2.26 | H | C≡CCMe₃ | F | Me | Me | O |
| 2.27 | H | C≡CMe | H | Me | CF₃ | O |
| 2.28 | H | C≡CMe | H | Me | CF₂H | O |
| 2.29 | H | C≡CMe | F | Me | Me | O |
| 2.30 | H | C≡CH | H | CF₂H | CF₂H | O |
| 2.31 | H | C≡CH | H | CF₂H | CF₃ | O |
| 2.32 | H | C≡CH | H | Me | CH₂F | O |
| 2.33 | H | C≡CSiMe₃ | H | Me | CH₂F | O |
| 2.34 | H | C≡C(cyclopropyl) | H | Me | CF₃ | O |
| 2.35 | H | C≡C(cyclopropyl) | H | Me | CHF₂ | O |
| 2.36 | H | C≡CF | H | Me | CF₃ | O |
| 2.37 | H | C≡CF | H | Me | CF₂H | O |
| 2.38 | H | C≡CF | F | Me | Me | O |
| 2.39 | H | C≡CCF₂Cl | H | Me | CF₃ | O |
| 2.40 | H | C≡CCF₂Cl | H | Me | CF₂H | O |
| 2.41 | H | C≡CCF₂Cl | F | Me | Me | O |
| 2.42 | H | C≡CCF₂H | H | Me | CF₃ | O |
| 2.43 | H | C≡CCF₂H | H | Me | CF₂H | O |
| 2.44 | H | C≡CCF₂H | F | Me | Me | O |
| 2.45 | H | C≡CCF₂Br | H | Me | CF₃ | O |
| 2.46 | H | C≡CCF₂Br | H | Me | CF₂H | O |
| 2.47 | H | C≡CCF₂Br | F | Me | Me | O |
| 2.48 | H | C≡CCH₂F | H | Me | CF₃ | O |
| 2.49 | H | C≡CCH₂F | H | Me | CF₂H | O |
| 2.50 | H | C≡CCH₂F | F | Me | Me | O |
| 2.51 | H | C≡CCH(Me)F | H | Me | CF₃ | O |
| 2.52 | H | C≡CCH(Me)F | H | Me | CF₂H | O |
| 2.53 | H | C≡CCH(Me)F | F | Me | Me | O |
| 2.54 | H | C≡CC(Me)₂F | H | Me | CF₃ | O |
| 2.55 | H | C≡CC(Me)₂F | H | Me | CF₂H | O |
| 2.56 | H | C≡CC(Me)₂F | F | Me | Me | O |
| 2.57 | H | C≡CCH₂C(Me)₃ | H | Me | CF₃ | O |
| 2.58 | H | C≡CCH₂C(Me)₃ | H | Me | CF₂H | O |
| 2.59 | H | C≡CCH₂C(Me)₃ | F | Me | Me | O |
| 2.60 | H | C≡CCH(Me)₂ | H | Me | CF₃ | O |
| 2.61 | H | C≡CCH(Me)₂ | H | Me | CF₂H | O |
| 2.62 | H | C≡CCH(Me)₂ | F | Me | Me | O |
| 2.63 | H | C≡CCH₂CH(Me)₂ | H | Me | CF₃ | O |
| 2.64 | H | C≡CCH₂CH(Me)₂ | H | Me | CF₂H | O |
| 2.65 | H | C≡CCH₂CH(Me)₂ | F | Me | Me | O |
| 2.66 | H | CH₂C≡CCMe₃ | H | Me | CF₃ | O |
| 2.67 | H | CH₂C≡CCMe₃ | H | Me | CF₂H | O |
| 2.68 | H | CH₂C≡CCMe₃ | F | Me | Me | O |
| 2.69 | H | CF₂C≡CCMe₃ | H | Me | CF₃ | O |
| 2.70 | H | CF₂C≡CCMe₃ | H | Me | CF₂H | O |
| 2.71 | H | CF₂C≡CCMe₃ | F | Me | Me | O |
| 2.72 | H | CF₂C≡CMe | H | Me | CF₃ | O |
| 2.73 | H | CF₂C≡CMe | H | Me | CF₂H | O |
| 2.74 | H | CF₂C≡CMe | F | Me | Me | O |
| 2.75 | H | CF₂C≡CH | H | Me | CF₃ | O |
| 2.76 | H | CF₂C≡CH | H | Me | CF₂H | O |
| 2.77 | H | CF₂C≡CH | F | Me | Me | O |
| 2.78 | H | CMe₂C≡CH | H | Me | CF₃ | O |
| 2.79 | H | CMe₂C≡CH | H | Me | CF₂H | O |
| 2.80 | H | CMe₂C≡CH | F | Me | Me | O |
| 2.81 | H | CHFC≡CH | H | Me | CF₃ | O |
| 2.82 | H | CHFC≡CH | H | Me | CF₂H | O |
| 2.83 | H | CHFC≡CH | F | Me | Me | O |
| 2.84 | H | CHMeC≡CH | H | Me | CF₃ | O |
| 2.85 | H | CHMeC≡CH | H | Me | CF₂H | O |
| 2.86 | H | CHMeC≡CH | F | Me | Me | O |
| 2.87 | H | CH(CF₃)C≡CH | H | Me | CF₃ | O |
| 2.88 | H | CH(CF₃)C≡CH | H | Me | CF₂H | O |
| 2.89 | H | CH(CF₃)C≡CH | F | Me | Me | O |
| 2.90 | H | C≡C (1-F-cyclopentyl) | H | Me | CF₃ | O |
| 2.91 | H | C≡C (1-F-cyclopentyl) | H | Me | CHF₂ | O |
| 2.92 | H | C≡CCH₂OMe | H | Me | CF₃ | O |
| 2.93 | H | C≡CCH₂OMe | H | Me | CF₂H | O |
| 2.94 | H | C≡CCH₂OMe | F | Me | Me | O |
| 2.95 | H | C≡CCMe₂OMe | H | Me | CF₃ | O |
| 2.96 | H | C≡CCMe₂OMe | H | Me | CF₂H | O |
| 2.97 | H | C≡CCMe₂OMe | F | Me | Me | O |
| 2.98 | H | C≡CCF₂Me | H | Me | CF₃ | O |
| 2.99 | H | C≡CCF₂Me | H | Me | CF₂H | O |
| 2.100 | H | C≡CCF₂Me | F | Me | Me | O |
| 2.101 | H | C≡CC(Me)=CH₂ | H | Me | CF₃ | O |
| 2.102 | H | C≡CC(Me)=CH₂ | H | Me | CF₂H | O |

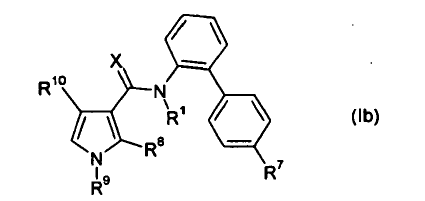

Table 2 provides 102 compounds of formula (Ib) wherein R¹, R⁷, R⁸, R⁹, R¹⁰ and X are as defined in Table 2.

**Table 3**

| Compound No. | R¹ | R⁷ | R⁹ | R¹⁰ | X |
|---|---|---|---|---|---|
| 3.01 | H | C≡CH | Me | CF₃ | O |
| 3.02 | H | C≡CH | Me | CF₂H | O |
| 3.03 | H | C≡CH | Me | Me | O |
| 3.04 | H | C≡CH | CH₂OMe | CF₃ | O |
| 3.05 | H | C≡CSiMe₃ | Me | CF₃ | O |
| 3.06 | H | C≡CSiMe₃ | Me | CF₂H | O |
| 3.07 | H | C≡CSiMe₃ | Me | Me | O |
| 3.08 | H | C≡CCl | Me | CF₃ | O |
| 3.09 | H | C≡CCl | Me | CF₂H | O |
| 3.10 | H | C≡CCl | Me | Me | O |
| 3.11 | H | C≡CBr | Me | CF₃ | O |
| 3.12 | H | C≡CBr | Me | CF₂H | O |
| 3.13 | H | C≡CBr | Me | Me | O |
| 3.14 | H | C≡CCF₃ | Me | CF₃ | O |
| 3.15 | H | C≡CCF₃ | Me | CF₂H | O |
| 3.16 | H | C≡CCF₃ | Me | Me | O |
| 3.17 | H | CH₂C≡CH | Me | CF₃ | O |
| 3.18 | H | CH₂C≡CH | Me | CF₂H | O |
| 3.19 | H | CH₂C≡CH | Me | Me | O |
| 3.20 | H | CH₂C≡CH | CH₂OMe | CF₃ | O |
| 3.21 | H | CH₂C≡CSiMe₃ | Me | CF₃ | O |
| 3.22 | H | CH₂C≡CSiMe₃ | Me | CF₂H | O |
| 3.23 | H | CH₂C≡CSiMe₃ | Me | Me | O |
| 3.24 | H | C≡CCMe₃ | Me | CF₃ | O |
| 3.25 | H | C≡CCMe₃ | Me | CF₂H | O |
| 3.26 | H | C≡CCMe₃ | Me | Me | O |
| 3.27 | H | C≡CMe | Me | CF₃ | O |
| 3.28 | H | C≡CMe | Me | CF₂H | O |
| 3.29 | H | C≡CMe | Me | Me | O |
| 3.30 | H | C≡CH | CF₂H | CF₂H | O |
| 3.31 | H | C≡CH | CF₂H | CF₃ | O |
| 3.32 | H | C≡CH | Me | CH₂F | O |
| 3.33 | H | C≡CSiMe₃ | Me | CH₂F | O |
| 3.34 | H | C≡C(cyclopropyl) | Me | CF₃ | O |
| 3.35 | H | C≡C(cyclopropyl) | Me | CHF₂ | O |
| 3.36 | H | C≡CF | Me | CF₃ | O |
| 3.37 | H | C≡CF | Me | CF₂H | O |
| 3.38 | H | C≡CF | Me | Me | O |
| 3.39 | H | C≡CCF₂Cl | Me | CF₃ | O |
| 3.40 | H | C≡CCF₂Cl | Me | CF₂H | O |
| 3.41 | H | C≡CCF₂Cl | Me | Me | O |
| 3.42 | H | C≡CCF₂H | Me | CF₃ | O |
| 3.43 | H | C≡CCF₂H | Me | CF₂H | O |
| 3.44 | H | C≡CCF₂H | Me | Me | O |
| 3.45 | H | C≡CCF₂Br | Me | CF₃ | O |
| 3.46 | H | C≡CCF₂Br | Me | CF₂H | O |
| 3.47 | H | C≡CCF₂Br | Me | Me | O |
| 3.48 | H | C≡CCH₂F | Me | CF₃ | O |
| 3.49 | H | C≡CCH₂F | Me | CF₂H | O |
| 3.50 | H | C≡CCH₂F | Me | Me | O |
| 3.51 | H | C≡CCH(Me)F | Me | CF₃ | O |
| 3.52 | H | C≡CCH(Me)F | Me | CF₂H | O |
| 3.53 | H | C≡CCH(Me)F | Me | Me | O |
| 3.54 | H | C≡CC(Me)₂F | Me | CF₃ | O |
| 3.55 | H | C≡CC(Me)₂F | Me | CF₂H | O |
| 3.56 | H | C≡CC(Me)₂F | Me | Me | O |
| 3.57 | H | C≡CCH₂C(Me)₃ | Me | CF₃ | O |
| 3.58 | H | C≡CCH₂C(Me)₃ | Me | CF₂H | O |
| 3.59 | H | C≡CCH₂C(Me)₃ | Me | Me | O |
| 3.60 | H | C≡CCH(Me)₂ | Me | CF₃ | O |
| 3.61 | H | C≡CCH(Me)₂ | Me | CF₂H | O |
| 3.62 | H | C≡CCH(Me)₂ | Me | Me | O |
| 3.63 | H | C≡CCH₂CH(Me)₂ | Me | CF₃ | O |
| 3.64 | H | C≡CCH₂CH(Me)₂ | Me | CF₂H | O |
| 3.65 | H | C≡CCH₂CH(Me)₂ | Me | Me | O |
| 3.66 | H | CH₂C≡CCMe₃ | Me | CF₃ | O |
| 3.67 | H | CH₂C≡CCMe₃ | Me | CF₂H | O |
| 3.68 | H | CH₂C≡CCMe₃ | Me | Me | O |
| 3.69 | H | CF₂C≡CCMe₃ | Me | CF₃ | O |
| 3.70 | H | CF₂C≡CCMe₃ | Me | CF₂H | O |
| 3.71 | H | CF₂C≡CCMe₃ | Me | Me | O |
| 3.72 | H | CF₂C≡CMe | Me | CF₃ | O |
| 3.73 | H | CF₂C≡CMe | Me | CF₂H | O |
| 3.74 | H | CF₂C≡CMe | Me | Me | O |
| 3.75 | H | CF₂C≡CH | Me | CF₃ | O |
| 3.76 | H | CF₂C≡CH | Me | CF₂H | O |
| 3.77 | H | CF₂C≡CH | Me | Me | O |
| 3.78 | H | CMe₂C≡CH | Me | CF₃ | O |
| 3.79 | H | CMe₂C≡CH | Me | CF₂H | O |
| 3.80 | H | CMe₂C≡CH | Me | Me | O |
| 3.81 | H | CHFC≡CH | Me | CF₃ | O |
| 3.82 | H | CHFC≡CH | Me | CF₂H | O |
| 3.83 | H | CHFC≡CH | Me | Me | O |
| 3.84 | H | CHMeC≡CH | Me | CF₃ | O |
| 3.85 | H | CHMeC≡CH | Me | CF₂H | O |
| 3.86 | H | CHMeC≡CH | Me | Me | O |
| 3.87 | H | CH(CF₃)C≡CH | Me | CF₃ | O |
| 3.88 | H | CH(CF₃)C≡CH | Me | CF₂H | O |
| 3.89 | H | CH(CF₃)C≡CH | Me | Me | O |
| 3.90 | H | C≡C (1-F-cyclopentyl) | Me | CF₃ | O |
| 3.91 | H | C≡C (1-F-cyclopentyl) | Me | CHF₂ | O |
| 3.92 | H | C≡CCH₂OMe | Me | CF₃ | O |
| 3.93 | H | C≡CCH₂OMe | Me | CF₂H | O |
| 3.94 | H | C≡CCH₂OMe | Me | Me | O |
| 3.95 | H | C≡CCMe₂OMe | Me | CF₃ | O |
| 3.96 | H | C≡CCMe₂OMe | Me | CF₂H | O |
| 3.97 | H | C≡CCMe₂OMe | Me | Me | O |
| 3.98 | H | C≡CCF₂Me | Me | CF₃ | O |
| 3.99 | H | C≡CCF₂Me | Me | CF₂H | O |
| 3.100 | H | C≡CCF₂Me | Me | Me | O |
| 3.101 | H | C≡CC(Me)=CH₂ | Me | CF₃ | O |
| 3.102 | H | C≡CC(Me)=CH₂ | Me | CF₂H | O |
| 3.103 | H | C≡ CCMe₂OH | Me | CF₃ | O |
| 3.104 | H | C≡ CCH₂CH₃ | Me | CF₂H | O |
| 3.105 | H | C≡ CCH₂CH₃ | Me | Me | O |
| 3.106 | H | C≡ CCH₂CH₃ | Me | CF₃ | O |
| 3.107 | H | C≡ CCF=CF₂ | Me | CF₃ | O |
| 3.108 | H | C≡ CCF=CF₂ | Me | CF₂H | O |
| 3.109 | H | C≡ CCHFCl | Me | CF₃ | O |
| 3.110 | H | C≡ CCHFCl | Me | CF₂H | O |
| 3.111 | H | C≡ CCHFCl | Me | Me | O |
| 3.112 | H | C≡ CCF₂CH₂CH₃ | Me | CF₃ | O |
| 3.113 | H | C≡ CCF₂CH₂CH₃ | Me | CF₂H | O |
| 3.1,14 | H | C≡ CCF₂CH₂CH₃ | Me | Me | O |
| 3.115 | H | C≡ CCHFCH₂CH₃ | Me | CF₃ | O |
| 3.116 | H | C≡ CCHFCH₂CH₃ | Me | CF₂H | O |
| 3.117 | H | C≡ CCHFCH₂CH₃ | Me | Me | O |
| 3.118 | H | C≡ CCF(CF₃)₂ | Me | CF₃ | O |
| 3.119 | H | C≡ CCF(CF₃)₂ | Me | CF₂H | O |
| 3.120 | H | C≡ CCF(CF₃)₂ | Me | Me | O |
| 3.121 | H | C≡ CC₂F₅ | Me | CF₃ | O |
| 3.122 | H | C= CC₂F₅ | Me | CF₂H | O |
| 3.123 | H | C≡ CC₂F₅ | Me | Me | O |

Table 3 provides 123 compounds of formula (Ic): 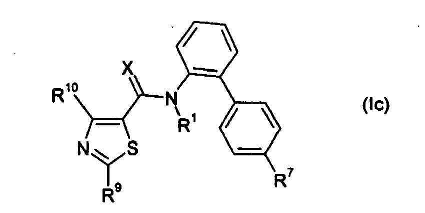 wherein R¹, R⁷, R⁹, R¹⁰ and X are as defined in Table 3.

Table 3 provides 123 compounds of formula (IcA) wherein R¹, R⁷, R⁹, R¹⁰ and X are as defined in Table 3.

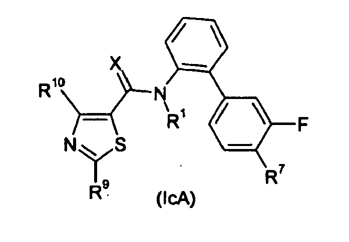

Table 3 provides 123 compounds of formula (IcB) wherein R¹, R⁷, R⁹, R¹⁰ and X are as defined in Table 3.

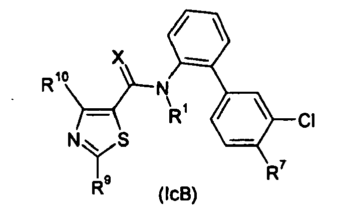

Table 3 provides 123 compounds of formula (IcC) wherein R¹, R⁷, R⁹, R¹⁰ and X are as defined in Table 3 .

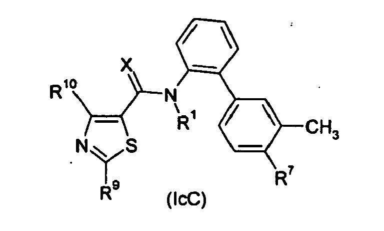

Table 3 provides 123 compounds of formula (IcD) wherein R¹, R⁷, R⁹, R¹⁰ and X are as defined in Table 3.

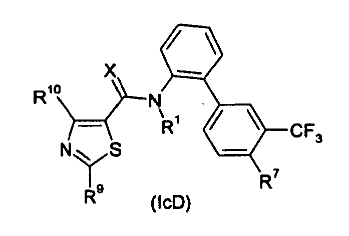

Table 3 provides 123 compounds of formula (IcE) wherein R¹, R⁷, R⁹, R¹⁰ and X are as defined in Table 3 .

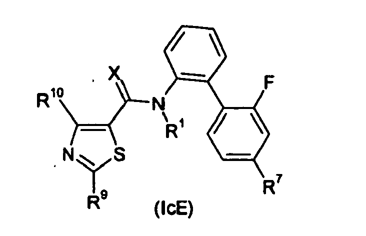

Table 3 provides 123 compounds of formula (IcF) wherein R¹, R⁷, R⁹, R¹⁰ and X are as defined in Table 3 .

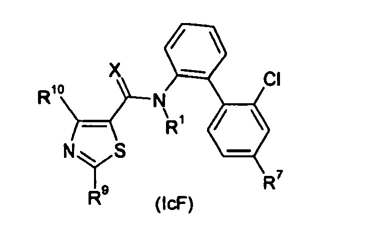

Table 3 provides 123 compounds of formula (IcG) wherein R¹, R⁷, R⁹, R¹⁰ and X are as defined in Table 3 .

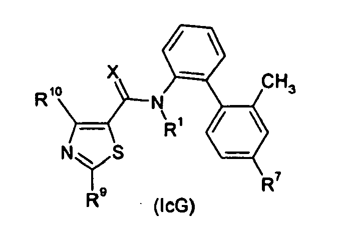

Table 4 provides 43 compounds of formula (II) where R², R³, R⁴ and R⁵ are each hydrogen; n is 0; and R¹ and R⁷ are as defined in Table 4.

**Table 4**

| Compound No. | R¹ | R⁷ |
|---|---|---|
| 4.01 | H | C≡CH |
| 4.02 | H | C≡ CSiMe₃ |
| 4.03 | H | C≡ CCF₃ |
| 4.04 | H | C≡ CCl |
| 4.05 | H | CH₂C≡ CH |
| 4.06 | H | C≡ CCMe₃ |
| 4.07 | H | C≡ C(cyclopropyl) |
| 4.08 | H | C≡ CBr |
| 4.09 | H | CH₂C≡ CSiMe₃ |
| 4.10 | H | C≡ CMe |
| 4.11 | H | C≡CF |
| 4.12 | H | C≡ CCF₂Cl |
| 4.13 | H | C≡ CCF₂H |
| 4.14 | H | C≡ CCF₂Br |
| 4.15 | H | C≡ CCH₂F |
| 4.16 | H | C≡ CCH(Me)F |
| 4.17 | H | C≡ CC(Me)₂F |
| 4.18 | H | C≡ CCH₂C(Me)₃ |
| 4.19 | H | C≡ CCH(Me)₂ |
| 4.20 | H | C≡CCH₂CH(Me)₂ |
| 4.21 | H | CH₂C≡ CCMe₃ |
| 4.22 | H | CF₂C≡ CCMe₃ |
| 4.23 | H | CF₂C≡ CMe |
| 4.24 | H | CF₂C≡ CH |
| 4.25 | H | CMe₂C≡ CH |
| 4.26 | H | CHFC≡ CH |
| 4.27 | H | CHMeC≡ CH |
| 4.28 | H | CH(CF₃)C≡ CH |
| 4.29 | H | C≡ C(1-F-cyclopentyl) |
| 4.30 | H | C≡ CCH₂OMe |
| 4.31 | H | C≡ CCMe₂OMe |
| 4.32 | H | C≡ CCMe₂OCOMe |
| 4.33 | H | C≡ CCF₂Me |
| 4.34 | H | C≡ CC(Me)CH₂ |
| 4.35 | H | C≡ CCH₂SiMe₃ |
| 4.36 | H | C≡ CSiMe₂CMe₃ |
| 4.37 | H | C≡ CCMe₂OH |
| 4.38 | H | C≡ CCH₂CH₃ |
| 4.39 | H | C≡ CCF=CF₂ |
| 4.40 | H | C≡ CCHFCl |
| 4.41 | H | C≡ CCF₂CH₂CH₃ |
| 4.42 | H | C≡ CCHFCH₂CH₃ |
| 4.43 | H | C≡ CCF(CF₃)₂ |

Table 7 provides 1 compound of formula (III) where R², R³, R⁴ and R⁵ are each hydrogen; n is 0; and Hal and R⁷ are as defined in Table 7.

**Table 5**

| Compound Number | R⁷ | Hal |
|---|---|---|
| 7.01 | C≡CH | Br |

Throughout this description, temperatures are given in degrees Celsius; "NMR" means nuclear magnetic resonance spectrum; MS stands for mass spectrum; M⁺-1 or M⁺+1 are signals in the mass spectrum respectively corresponding to the molecular weight minus 1 or the molecular weight plus 1; and "%" is percent by weight, unless corresponding concentrations are indicated in other units.

The following abbreviations are used throughout this description:

| | |
|---|---|
| m.p. = melting point | b.p.= boiling point. |
| s = singlet | br = broad |
| d = doublet | dd = doublet of doublets |
| t = triplet | q = quartet |
| m = multiplet | ppm = parts per million |

Table 8 shows selected melting point, selected molecular ion and selected NMR data, all with CDCl₃ as the solvent (unless otherwise stated; if a mixture of solvents is present, this is indicated as, for example, (CDCl₃ / d₆-DMSO)), (no attempt is made to list all characterising data in all cases) for compounds of Tables 1 to 5. Unless otherwise stated, the data relate to a cis/trans mixture of each compound.

**Table 8**

| Compound Number | ¹H-NMR data: (ppm/multiplicity/number of Hs) or MS-data | m.p. / (°C) |
|---|---|---|
| 1.01 | | 169-170 |
| 1.02 | | 132-135 |
| 1.05 | | 147-150 |
| 1.06 | | >200 |
| 1.08 | | 195-197 |
| 1.09 | | 139-144 |
| 1.11 | | 193-194 |
| 1.12 | | 120-125 |
| 1.14 | | 207-209 |
| 1.15 | | 210-212 |
| 1.17 | | 186-190 |
| 1.18 | | 137-139 |
| 1.24 | | 139-143 |
| 1.25 | 406 (M⁺-1) | 193-196 |
| 1.27 | 382(M⁺-1) | 221-223 |
| 1.28 | 364 (M⁺-1) | 205-208 |
| 1.34 | | 208-210 |
| 1.35 | | 202-205 |
| 1.39 | | 198-198.5 |
| 1.40 | | 184-184.5 |
| 1.42 | | 206-207 |
| 1.43 | | 200-201 |
| 1.48 | | 205-206 |
| 1.49 | | 198-199 |
| 1.51 | | 197-198 (decomposition) |
| 1.52 | | 176-177 |
| 1.54 | | 120-121 |
| 1.55 | | 128-129 |
| 1.60 | | 155-158 |
| 1.61 | | 142-143 |
| 1.63 | | 150-153 |
| 1.64 | | 132-140 |
| 1.91 | | 132-133 |
| 1.92 | | 167-169 |
| 1.93 | | 163-165 |
| 1.95 | | 141-142.5 |
| 1.96 | | 155-156 |
| 1.99 | | 149-150 |
| 1.102 | | 143-145 |
| 1.103 | | 159-162 |
| 1.104 | | 156-161 |
| 1.105 | | 163-165 |
| 1.107 | | 139-140 |
| 1.108 | | 187-187.5 |
| 1.110 | | 177-178 |
| 2.01 | | 145-148 |
| 2.05 | | 148-154 |
| 2.24 | | 160-165 |
| 3.01 | | 145-147 |
| 3.05 | | 103-105 |
| 3.08 | | 122-126 |
| 3.14 | | 160-165 |
| 3.24 | | 129-133 |
| 3.27 | | 159-163 |
| 3.51 | | 133-134 |
| 3.54 | | 127.7-129 |
| 3.63 | | 117-119 |
| 3.90 | | 140-142 |
| 3.95 | 1.55(s,6); 2.7(s,3); 3.4(s,3); 7.2-7.3(m,4); 7.4(m,1); 7.5(d,2); 7.55(br,1); 8.3(d,1) | |
| 3.98 | | 141-142 |
| 3.103 | | 140-143 |
| 4.01 | | 111-115 |
| 4.02 | 0.05(s,9); 6.5-6.7(d+t,2); 6.8-7.1(t+t,2); 7.2-7.5(m,4) | |
| 4.03 | 262(M+H⁺); 303(M + MeCN + H⁺); | |
| 4.04 | | 92-98 |
| 4.05 | 208(M+H⁺); 249(M + MeCN + H⁺); | |
| 4.06 | | 66-69 |
| 4.07 | 0.8-0.9(m,4); 1.4-1.5(m,2); 3.7(br,2); 6.7-6.8(m,2); 7.1-7.2(m,2); 7.3-7.5(m,4) | |
| 4.08 | 3.8(br,2); 6.75-6.9(m,2); 7.1-7.2(m,2); 7.35-7.55(m,4) | |
| 4.10 | | 108-112 |
| 4.12 | 3.75(br,2); 6.8(m,2); 7.1-7.25(m,2); 7.5-7.7(m,4) ¹⁹F : -35.9 | |
| 4.13 | 3.8(br,2); 6.4(t,1); 6.75-6.9(m,2); 7.1-7.25(m,2); 7.4-7.56(m,4); ¹⁹F : -105.7 | |
| 4.15 | | 69-71 |
| 4.16 | 1.7(d ofd,3); 3.8(br,2); 5.5(d of quartetts,1); 6.75-6.9(m,2); 7.05-7.2(m,2); 7.4-7.6(m,4) ¹⁹F : -165.6 | |
| 4.17 | 1.75(d,6); 3.8(br,2); 6.8(m,2); 7.15(m,2); 7.4-7.6(m,4) ¹⁹F : -126.0 | |
| 4.19 | 1.3(d,6); 3.7(br,2); 6.7-6.8(d+t,2); 7.15(m,2); 7.3-7.5(m,4) | |
| 4.20 | 1.05(d,6); 1.9(m,1); 2.35(d,2); 3.75(br,2); 6.75-6.9(m,2); 7.1-7.2(m,2); 7.35-7.5(m,4) | |
| 4.29 | 1.7-2.5(m,8); 3.7(br,2); 6.7-6.8(m,2); 7.05-7.15(m,2); 7.35.7.5(m,4); 7.1-7.2(m,2); 7.4-7.6(m,4) | |
| 4.30 | 3.45(s,3); 3.8(br,2); 4.35(s,2); 6.7-6.8(d+t,2); 7.1-7.25(m,2); 7.4-7.6(m,4) | |
| 4.32 | 2.0(t,3); 3.75(br,2); 6.75-6.85(m,2); 7.1-7.2(m,2); 7.4-7.6(m,4) | |
| 4.33 | 2.0(s,3); 3.75(br,2); 5.3(narrow m,1); 5.4 (s,1); 6.75-6.85(m,2); 7.1-7.2(m,2); 7.4-7.55(m,4) | |
| 4.34 | 0.0(s,9); 1.6(s,2);6.6(d,1);6.65(t,1); 7.0(m,2); 7.2-7.4(m,4) | |
| 4.35 | 0.0(s,6); 0.8(s,9); 4.25(very broad,2);6.65-6.75(m,2); 6.9-7.05(m,2); 7.2-7.4(m,4) | |
| 4.36 | 252 (M + H⁺) | |
| 5.19 | | 131-134 |
| 5.20 | | Amorphous |
| 16.7 | | 63-64 |

Table 5 provides 36 compounds of formulas 1(m) where R,X and Het are as defined in Table 5.

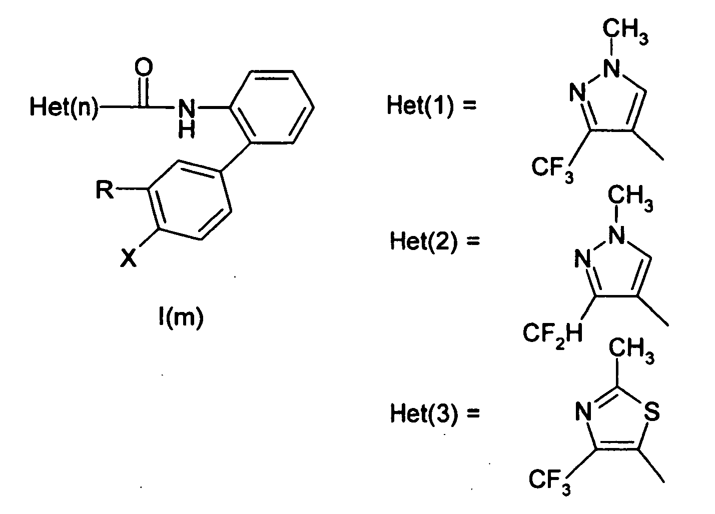

**Table 5**

| Compound No. | X | R | Het |
|---|---|---|---|
| 5.1 | H | C≡ CH | Het(1) |
| 5.2 | H | C≡ CH | Het(2) |
| 5.3 | H | C≡ CH | Het(3) |
| 5.4 | Cl | C≡ CH | Het(1) |
| 5.5 | Cl | C≡ CH | Het(2) |
| 5.6 | Cl | C≡ CH | Het(3) |
| 5.7 | F | C≡ CH | Het(1) |
| 5.08 | F | C≡ CH | Het(2) |
| 5.09 | F | C≡ CH | Het(3) |
| 5.10 | H | C≡ CMe | Het(1) |
| 5.11 | H | C≡ CMe | Het(2) |
| 5.12 | H | C≡ CMe | Het(3) |
| 5.13 | F | C≡ CMe | Het(1) |
| 5.14 | F | C≡ CMe | Het(2) |
| 5.15 | F | C≡ CMe | Het(3) |
| 5.16 | Cl | C≡ CMe | Het(1) |
| 5.17 | Cl | C≡ CMe | Het(2) |
| 5.18 | Cl | C≡ CMe | Het(3) |
| 5.19 | H | C≡ CCMe₃ | Het(1) |
| 5.20 | H | C≡ CCMe₃ | Het(2) |
| 5.21 | H | C≡ CCMe₃ | Het(3) |
| 5.22 | Cl | C≡ CCMe₃ | Het(1) |
| 5.23 | Cl | C≡ CCMe₃ | Het(2) |
| 5.24 | Cl | C≡CCMe₃ | Het(3) |
| 5.25 | F | C≡CCMe₃ | Het(1) |
| 5.26 | F | C≡CCMe₃ | Het(2) |
| 5.27 | F | C≡CCMe₃ | Het(3) |
| 5.28 | H | CH=CClCF₃ | Het(1) |
| 5.29 | H | CH=CClCF₃ | Het(2) |
| 5.30 | H | CH=CClCF₃ | Het(3) |
| 5.31 | Cl | CH=CClCF₃ | Het(1) |
| 5.32 | Cl | CH=CClCF₃ | Het(2) |
| 5.33 | Cl | CH=CClCF₃ | Het(3) |
| 5.34 | F | CH=CClCF₃ | Het(1) |
| 5.35 | F | CH=CClCF₃ | Het(2) |
| 5.36 | F | CH=CClCF₃ | Het(3) |

Table 6 provides 9 compounds of formulas II(m) where R and X are as defined in Table 6:

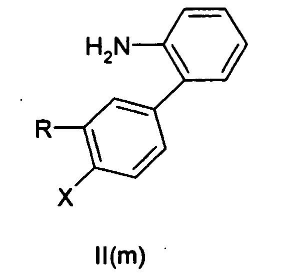

**Table 6**

| Compound No. | X | R |
|---|---|---|
| 6.1 | H | C≡ CH |
| 6.2 | Cl | C≡ CH |
| 6.3 | F | C≡ CH |
| 6.4 | H | C≡ CMe |
| 6.5 | F | C≡ CMe |
| 6.6 | Cl | C≡ CMe |
| 6.7 | H | C≡ CCMe₃ |
| 6.8 | Cl | C≡ CCMe₃ |
| 6.9 | F | C≡ CCMe₃ |

The compounds according to the present invention may be prepared according to the following reaction schemes, in which, unless otherwise stated, the definition of each variable is as defined above for a compound of formula (I).

There are a number of alternative methods for preparing a compound of formula (I).

### Method A

A compound of formula (I) may be prepared by reacting a compound of formula (II) with a compound of formula Het-C(=O)OR' [where R' is C₁₋₅ alkyl] in the presence of strong base [for example NaH or sodium hexamethyldisilazane], in a dry polar solvent [preferably THF] and at a temperature between -10°C and the boiling point of the solvent [preferably at ambient temperature]. The article by J.Wang et al, Synlett 2001,1485 provides details of analogous preparations.

### Method B

A compound of formula (I) may be prepared by reacting a compound of formula (II) with a compound of formula Het-C(=O)R" [where R" is OH or a leaving group, such as Cl, Br, F or OC(=O)C₁₋₄ alkyl] in an inert organic solvent [such as ethylacetate, dichloromethane, dioxane or DMF] and at a temperature between -10°C and the boiling point of the solvent [preferably at ambient temperature]. If R" is OH, the reaction is carried out in the presence of an activating agent [for example BOP-Cl] and two equivalents of a base [such as a tertiary amine, an inorganic carbonate or a hydrogen carbonate]. Alternatively, if R" is a leaving group, the reaction is carried out in the presence of at least one equivalent of base [such as pyridine, a tertiary amine, an inorganic carbonate or a hydrogen carbonate].

### Method C

A compound of formula (I) may be prepared by reacting a compound of formula (III) [where Hal is preferably bromo or iodo] with a compound of formula Het-C(=O)NH₂ in the presence of a Cu(I) compound and an aprotic solvent [such as a cyclic ether, for example dioxane] at an elevated temperature and preferably at reflux . The preferred conditions are Cul used at 2% to 100%mole/mole, relative to the compound of formula (III), in the presence of a 1,2-diamine as a ligand-forming substance (such as 1,2-diamino cyclohexane or ethylene diamine) and at least 1 equivalent of a base (such as an alkali carbonate or an alkali phosphate. The article by A.Klapars et al. J.Am.Chem.Soc. 123,7727 (2001) provides details of analogous preparations.

### Method D

A compound of formula (I) may be prepared by conversion of a compound of formula (IV) 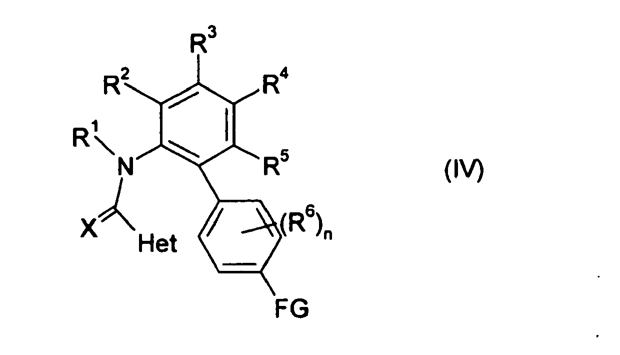 [where FG is a functional group which is convertible to R⁷ in one or more synthetic steps]. Functional group interconversions are standard procedures for a person skilled in the art. There are many methods described in the literature, which can be used as such or with modifications according to the functionalities present; Table A gives literature references (some of which also cite further appropriate references) which are specifically relevant to the preparation of a compound of formula (I) by the interconversion of FG to R⁷. It is apparent to the person skilled in the art that the literature examples given in Table A are not necessarily limited to the preparation of the specifically mentioned R⁷ but can be also applied by analogy to the preparation of other structurally related R⁷

**Table A**

| Reference | FG | R⁷ |
|---|---|---|
| Tetrahedron 58, 1491 (2002) | CHO | C≡ CBr |
| Bull.Chem.Soc.Jap. 62,1352 | CH=CClCF₃ | C≡CCF₃ |
| | CH=CFCF₂Cl | C≡ CCF₂Cl |
| J.Org.Chem. 54, 5856 (1989) | Hal or triflate | C≡ CH |
| J.Am.Chem.Soc. 109,2138 (1987) | | C≡ CSiMe₃ |
| Tetrahedron 45,6511 (1989) | | C≡ CCH₃ |
| J.Orgmet.Chem.549,127 (1997) | | C≡ CCMe₃ |
| Tetrahedron 56, 10075 (2000) | | C≡ CCH₂OH |
| Tetrahedron Asymmetry 6, 245 (1995) | | C≡ CCHMeOH |
| | | C≡ CCMe₂OH |
| | | C≡ CCHO |
| | | C≡ CC(O)Me |
| J.Org.Chem. 32, 1674 (1967) | C≡ CCH₃ | CH₂C≡ CH |
| Synth.Comm.1989,561 | CHO | C≡ CH |
| | CH₂CHO | CH₂C≡ CH |
| Synthesis 1996, 1494 | CHO | C≡ CCl |
| J.Org.Chem.49, 294 (1984) | | C≡ CH |
| | | C≡ CBr |
| J.Am.Chem.Soc. 123,4155 (2001) | CH₂Br | CH₂C≡ CSiMe₃ |
| Inorg.Chim.Acta 296, 37 (1999) | Hal | CH₂C≡ CMe₃ |
| DE 4417441 | C≡ CCH₂OH | C≡ CCH₂F |
| US3976691 | C≡ CCHMeOH | C≡ CCHMeF |
| J. Org. Chem. 64, 7048 (1999) | C≡ CCMe₂OH | C≡ CCMe₂F |
| | C≡ CCHO | C≡ CCHF₂ |
| | C≡ CC(O)Me | C≡ CCF₂Me |
| J.Chem.Soc. Perkin I 1994, 725 | C≡ CCH₂OH | C≡ CCH₂CF₃ |
| Synthesis 1997, 1489 | C≡ CH | C≡ CCF₂CF₃ |
| J.Fluorine.Chem.64, 61 (1993) | C≡ CH | C≡ CCHFCl |
| J.Am.Chem.Soc. 109, 3492 (1987) | | C≡ CCF₂Br |

There are a number of alternative methods for preparing a compound of formula (II), (III) or (IV).

### Method E - preparation of a compound of formula (II) or (III).

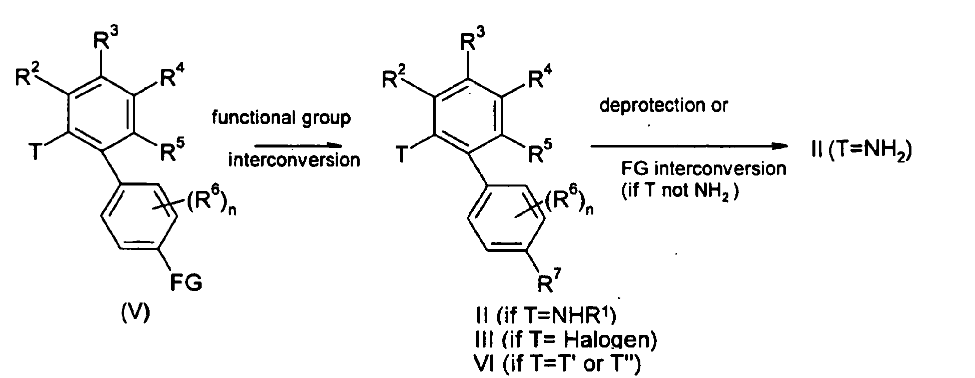

A compound of formula (II), (III) or (VI) may be prepared, by functional group interconversion, from a compound of formula (V) [where FG is as defined above for a compound of formula (IV) and T is either halogen, amino, NHR¹, a protected amino group T' (for example a carbamate, an amide, a cyclic imide, an N-alkyl-, N-alkenyl-, N-benzyl-, N-diphenylmethyl- or N-trityl-derivative, an imine derivative or an N-silyl- or N-disilyl-derivative) or a group T" (that is, a group which may be converted to NH₂ or NHR' by applying synthetic methodology described in the literature; T" being preferably azido, nitro, halogen, triflate, CONH₂, COOH, COCI or NCO)]. Starting from a compound of formula (V) the functional group FG may be converted to R⁷ by applying a method analogous to method E above. This conversion leads directly to a compound of formula (II) [when T is NHR₁], to a compound of formula (III) [when T is halogen (preferably chloro, bromo or iodo)] or to a compound of formula (VI) [when T is T' or T"].

In a second step a compound of formula (VI) or (II) [when R¹ is other than H] can be converted to a compound of formula (II) [where R¹ is H] by either applying the methods [that is, deprotection or conversion of T" to NH₂] as generically described above.

Examples of versatile values for T' plus methods for deprotection are given in T.W.Green and P.Wuts, Protective Groups in Organic Synthesis, 3rd edition (John Wiley & Sons 1999), Chapter 7.

Compilations of useful values for T" plus literature to convert T" into NH₂, T' or NHR' can be found in M.B. Smith, Compendium of Organic Synthetic Methods, Vols. 1-10, Chapter 7 (Wiley, Vol .10: 2002).

### Method F

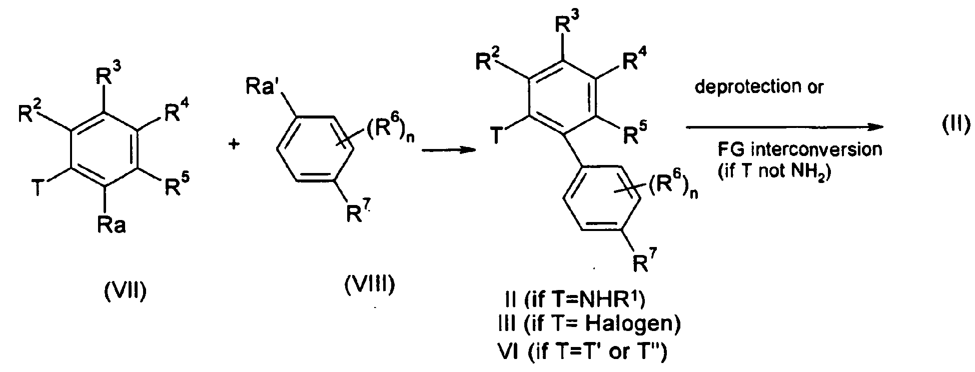

A compound of formula (II), (III) or (VI) may be prepared by a coupling reaction between a compound of formula (VII) and a compound of formula (VIII) [where Ra and Ra' are each, independently, halogen (preferably Cl, Br or I), triflate or a metal-containing functionality containing, for example, B, Sn, Mg, Zn or Cu as the metal; examples are B(OH)₂, esters of boronic acid (preferably esters derived from 1,2- or 1,3-diols), trialkyltin (preferably Sn(CH₃)₃ or Sn(nBu)₃), a halogen salt of Mg, a halogen salt of Zn or Cu. If either Ra or Ra' is a metal containing functionality, the other substituent must be halogen or triflate.

Such coupling reactions are widely known in the literature. Especially suitable are the Pd(0) ,Ni(0), or copper catalysed couplings which are well known to the person skilled in the art as Stille coupling, Suzuki coupling, Negishi coupling or Ullmann reaction. A comprehensive review of these reactions can be found in Metal-Catalysed Cross-Coupling Reactions; F.Diederich and P.Stang (eds.); Wiley-VCH; Weinheim 1998.

In a second step a compound of formula (VI) or (II) [when R¹ is other than H] can be converted to a compound of formula (II) [where R¹ is H] by either applying the methods [that is, deprotection or conversion of T" to NH₂] as generically described above.

Surprisingly, it has now been found that the novel compounds of formula (I) have, for practical purposes, a very advantageous spectrum of activities for protecting plants against diseases that are caused by fungi as well as by bacteria and viruses.

The compounds of formula (I) can be used in the agricultural sector and related fields of use as active ingredients for controlling plant pests. The novel compounds are distinguished by excellent activity at low rates of application, by being well tolerated by plants and by being environmentally safe. They have very useful curative, preventive and systemic properties and are used for protecting numerous cultivated plants. The compounds of formula I can be used to inhibit or destroy the pests that occur on plants or parts of plants (fruit, blossoms, leaves, stems, tubers, roots) of different crops of useful plants, while at the same time protecting also those parts of the plants that grow later e.g. from phytopathogenic microorganisms.

It is also possible to use compounds of formula (I) as dressing agents for the treatment of plant propagation material, in particular of seeds (fruit, tubers, grains) and plant cuttings (e.g. rice), for the protection against fungal infections as well as against phytopathogenic fungi occurring in the soil.

Furthermore the compounds according to present invention may be used for controlling fungi in related areas, for example in the protection of technical materials, including wood and wood related technical products, in food storage, in hygiene management, etc.

The compounds of formula (I) are, for example, effective against the phytopathogenic fungi of the following classes: Fungi imperfecti (e.g. Botrytis, Pyricularia, Helminthosporium, Fusarium, Septoria, Cercospora and Altemaria) and Basidiomycetes (e.g. Rhizoctonia, Hemileia, Puccinia). Additionally, they are also effective against the Ascomycetes classes (e.g. Venturia and Erysiphe, Podosphaera, Monilinia, Uncinula) and of the Oomycetes classes (e.g. Phytophthora, Pythium, Plasmopara). Outstanding activity has been observed against powdery mildew (Erysiphe spp.) and rust ( Puccinia spp.). Furthermore, the novel compounds of formula I are effective against phytopathogenic bacteria and viruses (e.g. against Xanthomonas spp, Pseudomonas spp, Erwinia amylovora as well as against the tobacco mosaic virus).

Within the scope of present invention, target crops to be protected typically comprise the following species of plants: cereal (wheat, barley, rye, oat, rice, maize, sorghum and related species); beet (sugar beet and fodder beet); pomes, drupes and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamomum, camphor) or plants such as tobacco, nuts, coffee, eggplants, sugar cane, tea, pepper, vines, hops, bananas and natural rubber plants, as well as ornamentals.

The compounds of formula (I) are used in unmodified form or, preferably, together with the adjuvants conventionally employed in the art of formulation. To this end they are conveniently formulated in known manner to emulsifiable concentrates, coatable pastes, directly sprayable or dilutable solutions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of formula (I) are normally used in the form of compositions and can be applied to the crop area or plant to be treated, simultaneously or in succession with further compounds. These further compounds can be e.g. fertilizers or micronutrient donors or other preparations which influence the growth of plants. They can also be selective herbicides as well as insecticides, fungicides, bactericides, nematicides, molluscicides or mixtures of several of these preparations, if desired together with further carriers, surfactants or application promoting adjuvants customarily employed in the art of formulation.

The compounds of formula (I) can be mixed with other fungicides, resulting in some cases in unexpected synergistic activities. Mixing components which are particularly preferred are azoles, such as azaconazole, BAY 14120, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, pyrifenox, prochloraz, propiconazole, simeconazole, tebuconazole, tetraconazole, triadimefon, triadimenol, triflumizole, triticonazole; pyrimidinyl carbinole, such as ancymidol, fenarimol, nuarimol; 2-amino-pyrimidines, such as bupirimate, dimethirimol, ethirimol; morpholines, such as dodemorph, fenpropidine, fenpropimorph, spiroxamine, tridemorph; anilinopyrimidines, such as cyprodinil, mepanipyrim, pyrimethanil; pyrroles, such as fenpiclonil, fludioxonil; phenylamides, such as benalaxyl, furalaxyl, metalaxyl, R-metalaxyl, ofurace, oxadixyl; benzimidazoles, such as benomyl, carbendazim, debacarb, fuberidazole, thiabendazole; dicarboximides, such as chlozolinate, dichlozoline, iprodione, myclozoline, procymidone, vinclozoline; carboxamides, such as carboxin, fenfuram, flutolanil, mepronil, oxycarboxin, thifluzamide; guanidines, such as guazatine, dodine, iminoctadine; strobilurines, such as azoxystrobin, kresoxim-methyl, metominostrobin, SSF-129, trifloxystrobin, picoxystrobin, BAS 500F (proposed name pyraclostrobin), BAS 520; dithiocarbamates, such as ferbam, mancozeb, maneb, metiram, propineb, thiram, zineb, ziram; N-halomethylthiotetrahydrophthalimides, such as captafol, captan, dichlofluanid, fluoromides, folpet, tolyfluanid; Cu-compounds, such as Bordeaux mixture, copper hydroxide, copper oxychloride, copper sulfate, cuprous oxide, mancopper, oxine-copper; nitrophenol-derivatives, such as dinocap, nitrothal-isopropyl; organo-p-derivatives, such as edifenphos, iprobenphos, isoprothiolane, phosdiphen, pyrazophos, tolclofos-methyl; various others, such as acibenzolar-S-methyl, anilazine, benthiavalicarb, blasticidin-S, chinomethionate, chloroneb, chlorothalonil, cyflufenamid, cymoxanil, dichlone, diclomezine, dicloran, diethofencarb, dimethomorph, SYP-LI90 (proposed name: flumorph), dithianon, ethaboxam, etridiazole, famoxadone, fenamidone, fenoxanil, fentin, ferimzone, fluazinam, flusulfamide, fenhexamid, fosetyl-aluminium, hymexazol, iprovalicarb, IKF-916 (cyazofamid), kasugamycin, methasulfocarb, metrafenone, nicobifen, pencycuron, phthalide, polyoxins, probenazole, propamocarb, pyroquilon, quinoxyfen, quintozene, sulfur, triazoxide, tricyclazole, triforine, validamycin, zoxamide (RH7281).

A preferred method of applying a compound of formula (I), or an agrochemical composition which contains at least one of said compounds, is foliar application. The frequency of application and the rate of application will depend on the risk of infestation by the corresponding pathogen. However, the compounds of formula I can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula I may also be applied to seeds (coating) by impregnating the seeds or tubers either with a liquid formulation of the fungicide or coating them with a solid formulation.

A formulation [that is, a composition containing the compound of formula (I)] and, if desired, a solid or liquid adjuvant, is prepared in a known manner, typically by intimately mixing and/or grinding the compound with extenders, for example solvents, solid carriers and, optionally, surface active compounds (surfactants).

The agrochemical formulations will usually contain from 0.1 to 99% by weight, preferably from 0.1 to 95% by weight, of the compound of formula 1, 99.9 to 1% by weight, preferably 99.8 to 5% by weight, of a solid or liquid adjuvant, and from 0 to 25% by weight, preferably from 0.1 to 25% by weight, of a surfactant.

Advantageous rates of application are normally from 5g to 2kg of active ingredient (a.i.) per hectare (ha), preferably from 10g to 1kg a.i./ha, most preferably from 20g to 600g a.i./ha. When used as seed drenching agent, convenient dosages are from 10mg to 1g of active substance per kg of seeds.

Whereas it is preferred to formulate commercial products as concentrates, the end user will normally use dilute formulations.

The following non-limiting Examples illustrate the above-described invention in more detail.

### EXAMPLE 1

This Example illustrates the preparation of Compound No. 1.01.

2-Amino-4'-ethinyl-biphenyl (0.30g) and 1-methyl-3-trifluoromethyl-4-chlorocarbonyl-pyrazol (0.33g) were combined in THF under cooling with ice and then pyridine (0.12ml) was added. After warming to ambient temperature the suspension was stirred for 3.5hours, poured into water and extracted twice with ethylacetate. Separation of the organic phase, drying with sodium sulfate and evaporation of the solvent and chromatographic purification on silica gel (solvent: hexane:ethylacetate 2:1) yielded 0.4g (70.2%) of Compound No. 1.01.

### EXAMPLE 2

This Example illustrates the preparation of Compound No. 2.01.

To 1-methyl-3-trifluoromethyl-4-pyrrol carboxylic acid (0.22g) dissolved in 10ml dichloromethane were added triethylamine (0.32ml) and 2-amino-4'-trimethylsilylethinyl-biphenyl (0.3g) and finally, under cooling with ice, bis(2-oxo-3-oxazolidinyl) chlorophosphinic acid (0.29g). After stirring for 18hours the solvents were removed under reduced pressure and the residue was taken up with ethylacetate. Washing with water and brine, drying with sodiumsulfate and evaporation of the solvent yielded 0.45g of a yellow oil which was chromatographed on silica gel (eluent: hexane:ethylacetate 2:1) to yield 0.13g (26%) of Compound No. 2.01.

### EXAMPLE 3

This Example illustrates the preparation of 2- amino-4'-(trimethylsilyl)ethinyl-biphenyl (Compound No.4.02) and 2-amino-4'-ethinyl-biphenyl (Compound No. 4.01) using a preparation according to Method E above.

To 2.5g 2-amino-4'-bromo-biphenyl (WO0264562) in piperidine (25ml) under nitrogen were added in sequence CuI (0.1g), bis(triphenylphosphino)palladium dichloride (0.35g) and trimethylsilylacetylene (2.8ml). The mixture was stirred for 22hours at room temperature and for a further 26hours at 60°C. After cooling the reaction mixture was diluted with water and extracted with ethylacetate. Then the organic phase was washed with water and dried over sodium sulfate. After evaporation of the solvents in vacuum the mixture was chromatographed on silica gel (hexane:ethylacetate 9:1) to yield 2- amino-4'-(trimethylsilyl)ethinyl-biphenyl (2g) (Compound No.4.02).

1.4g of this compound was dissolved in methanol (40ml) and potassium carbonate (0.9g) was added with cooling. The resultant suspension was stirred for 2hours, poured on ice-water and the precipitate formed was filtered off, washed thoroughly with water and dried to obtain 2-amino-4'-ethinyl-biphenyl (0.9g) (Compound No.4.01) as light tan crystals.

### EXAMPLE 4

This Example illustrates the preparation of 2-amino-4'(2,2-dichloro)ethylene-biphenyl and 2-amino-4'(chloroethinyl)-biphenyl (Compound No 4.04.)

a) Preparation of 2-nitro-4'(2,2-dichloro)ethylene-biphenyl.

To 2-nitro-4'formyl-biphenyl (2g) (WO 95 03290) (prepared by Pd-catalysed coupling of 2-bromonnitrobenzene with 4-formyl-phenyl-boronic acid) in ethanol (70ml) was added hydrazine hydrate (95%) (1.32g) and the resultant mixture was then refluxed for 5hours. The solvent was evaporated to dryness under reduced pressure, the residue was suspended in DMSO (30ml) and then ammonia (25%) (3ml) and freshly prepared CuCl (80mg) were sequentially added and finally tetrachlorometane (3.8g) was dropped in under cooling with water. The mixture was stirred at room temperature for 24hours and the resultant green suspension was poured into water, extracted with dichloromethane, washed with water and dried over sodium sulfate. Evaporation of the solvent and chromatography of the residue over silicagel (eluent:hexane:ethylacetate 4:1) yielded 2-nitro-4'(2,2-dichloro)ethylene-biphenyl (0.8g), m.p. 58-59°C.

b) Preparation of 2-amino-4'(2,2-dichloro)ethylene-biphenyl.

2-Nitro-4'(2,2-dichloro)ethylene-biphenyl (0.76g) from step (a) was dissolved in 50% ethanol (30ml) and heated to reflux. Then 2N HCl (0.3ml) in 50% ethanol (10ml) was added dropwise. The reaction mixture was held at reflux for 4hours, cooled to room temperature and filtered. The filtrate was neutralised with sodium bicarbonate, extracted twice with ethylacetate and the organic phase was dried over sodium sulfate. Evaporation of the solvent under reduced pressure yielded 2-amino-4'(2,2-dichloro)ethylene-biphenyl (0.62g) .

c) 2-Amino-4'(2,2-dichloro)ethylene-biphenyl (3g) was dissolved in 150ml dimethyl sulfoxide in which 0.9g KOH (85%, powder) has been suspended. The mixture was stirred over night at room temperature, diluted with excess of water and extracted twice with ethyl acetate and the organic phase was dried over sodium sulfate. Evaporation of the solvent under reduced pressure and chromatography of the residue over silicagel (eluent:hexane:ethylacetate 4:1) yielded 2.5 g 2-amino-4'(2,2-dichloro)ethylene-biphenyl as a tan coloured solid.

### FORMULATION EXAMPLES FOR COMPOUNDS OF FORMULA (I)

Working procedures for preparing formulations of the compounds of formula I such as Emulsifiable Concentrates, Solutions, Granules, Dusts and Wettable Powders are described in WO97/33890.

### BIOLOGICAL EXAMPLES: FUNGICIDAL ACTIONS

### Example B-1: Action against Puccinia recondita / wheat (Brownrust on wheat)

1 week old wheat plants cv. Arina are treated with the formulated test compound (0.02% active ingredient) in a spray chamber. One day after application, the wheat plants are inoculated by spraying a spore suspension (1x10⁵uredospores/ml) on the test plants. After an incubation period of 2 days at 20°C and 95%r.h. the plants are kept in a greenhouse for 8days at 20°C and 60%r.h. The disease incidence is assessed 10days after inoculation.

Infestation is prevented virtually completely (0-5% infestation) with each of compounds 1.01, 1.02, 1.05, 1.06, 1.08, 1.09, 1.11, 1.12, 1.14, 1.15, 1.18, 1.24, 1.25, 1.27, 1.28, 1.34, 1.35, 1.60, 1.61, 1.92, 1.93, 1.104, 1. 105, 2.01, 2.05, 2.24, 3.01, 3.05, 3.08, 3.14, 3.24, 3.27, 3.103, 5.19 and 5.20.

### Example B-2: Action against Podosphaera leucotricha / apple (Powdery mildew on apple)

5 week old apple seedlings cv. McIntosh are treated with the formulated test compound (0.02% active ingredient) in a spray chamber. One day after, the application apple plants are inoculated by shaking plants infected with apple powdery mildew above the test plants. After an incubation period of 12 days at 22°C and 60%r.h. under a light regime of 14/10hours (light/dark) the disease incidence is assessed.

Infestation is prevented virtually completely (0-5% infestation) with each of compounds. 1.01, 1.02, 1.05, 1.06, 1.08, 1.09, 1.11, 1.12, 1.14, 1.15, 1.24, 1.25, 1.28, 1.34, 1.35, 1.60, 1.61, 1.93, 1.104, 1. 105, 2.01, 2.05, 2.24, 3.01, 3.05, 3.08, 3.14, 3.24, 3.27, 3.103, 5.19 and 5.20.

### Example B-3: Action against Venturia inaequalis / apple (Scab on apple)

4 week old apple seedlings cv. McIntosh are treated with the formulated test compound (0.02% active ingredient) in a spray chamber. One day after application, the apple plants are inoculated by spraying a spore suspension (4x10⁵conidia/ml) on the test plants. After an incubation period of 4 days at 21°C and 95%r.h. the plants are placed for 4 days at 21°C and 60%r.h. in a greenhouse. After another 4 day incubation period at 21°C and 95%r.h. the disease incidence is assessed.

Infestation is prevented virtually completely (0-5% infestation) with each of compounds. 1.01, 1.02, 1.05, 1.06, 1.08, 1.09, 1.14, 1.15, 1.24, 1.25, 1.27, 1.28, 1.34, 1.35, 1.60, 1.61, 1.92, 1.104, 1. 105, 2.01, 2.05, 2.24, 3.01, 3.05, 3.08, 3.14, 3.24 and 3.27.

### Example B-4: Action against Erysiphe graminis / barley (Powdery mildew on barley)

1 week old barley plants cv. Regina are treated with the formulated test compound (0.02% active ingredient) in a spray chamber. One day after application, the barley plants are inoculated by shaking powdery mildew infected plants above the test plants. After an incubation period of 6 days at 20°C / 18°C (day/night) and 60%r.h. in a greenhouse the disease incidence is assessed.

Infestation is prevented virtually completely (0-5% infestation) with each of compounds 1.01, 1.02, 1.05, 1.06, 1.08, 1.09, 1.11, 1.12, 1.14, 1.15, 1.24, 1.25, 1.28, 1.34, 1.35, 1.60, 1.61, 1.104, 1. 105, 2.01, 2.05, 2.24, 3.01, 3.05, 3.08, 3.14, 3.24, 3.27 and 3.103.

### Example B-5: Action against Botrytis cinerea / grape (Botrytis on grapes)

5 week old grape seedlings cv. Gutedel are treated with the formulated test compound (0.02% active ingredient) in a spray chamber. Two days after application, the grape plants are inoculated by spraying a spore suspension (1x10⁶ conidia/ml) on the test plants. After an incubation period of 4 days at 21°C and 95%r.h. in a greenhouse the disease incidence is assessed.

Infestation is prevented virtually completely (0-5% infestation) with each of compounds 1.01, 1.02, 1.05 and 1.06.

### Example B-6: Action against Botrytis cinerea / tomato (Botrytis on tomatoes)

4 week old tomato plants cv. Roter Gnom are treated with the formulated test compound (0.02% active ingredient) in a spray chamber. Two days after application, the tomato plants are inoculated by spraying a spore suspension (1x10⁵conidia/ml) on the test plants. After an incubation period of 4 days at 20°C and 95%r.h. in a growth chamber the disease incidence is assessed.

Infestation is prevented virtually completely (0-5% infestation) with each of compounds 1.01, 1.02, 1.05, 1.06, 1.08, 1.09, 1.11, 1.12, 1.14, 1.15, 1.24, 1.25, 1.27, 1.28, 1.34, 1.35, 1.60, 1.61, 1.92, 1.93, 1.104, 1. 105, 2.01, 2.24, 3.01, 3.05, 3.08, 3.24, 3.27 and 3.103.

### Example B-7: Action against Septoria nodorum / wheat (Septoria leaf spot on wheat)

1 week old wheat plants cv. Arina are treated with the formulated test compound (0.02% active ingredient) in a spray chamber. One day after application, the wheat plants are inoculated by spraying a spore suspension (5x10⁵conidia/ml) on the test plants. After an incubation period of 1 day at 20°C and 95%r.h. the plants are kept for 10 days at 20°C and 60%r.h. in a greenhouse. The disease incidence is assessed 11 days after inoculation.

Infestation is prevented virtually completely (0-5% infestation) with each of compounds 1.01, 1.02, 1.05 and 1.06.

### Example B-8: Action against Helminthosporium teres / barley (Net blotch on barley)

1 week old barley plants cv. Regina are treated with the formulated test compound (0.02% active ingredient) in a spray chamber. Two days after application, the barley plants are inoculated by spraying a spore suspension (3x10⁴ conidia/ml) on the test plants. After an incubation period of 4 days at 20°C and 95%r.h. in a greenhouse the disease incidence is assessed.

Infestation is prevented virtually completely (0-5% infestation) with each of compounds 1.01, 1.02, 1.05, 1.06, 1.08, 1.09, 1.11, 1.12, 1.14, 1.15, 1.18, 1.24, 1.25, 1.27, 1.28, 1.34, 1.35, 1.60, 1.61, 1.92, 1.93, 1.104, 1. 105, 2.01, 2.05, 2.24, 3.01, 3.05, 3.08, 3.14, 3.24, 3.27, 3.103, 5.19 and 5.20.

### Example B-9: Action against Alternaria solani / tomato (Early blight on tomatoes)

4 week old tomato plants cv. Roter Gnom are treated with the formulated test compound (0.02% active ingredient) in a spray chamber. Two days after application, the tomato plants are inoculated by spraying a spore suspension (2x10⁵conidia/ml) on the test plants. After an incubation period of 3 days at 20°C and 95%r.h. in a growth chamber the disease incidence is assessed.

Infestation is prevented virtually completely (0-5% infestation) with each of compounds 1.01, 1.02, 1.05, 1.06, 1.09, 1.11, 1.12, 1.15, 1.25, 1.27, 1.28, 1.34, 1.35, 1.61, 1.104, 1. 105, 2.01, 3.01, 3.05, 3.08, 3.27 and 5.20.

### Example B-10: Action against Uncinula necator / grape (Powdery mildew on grapes)

5 week old grape seedlings cv. Gutedel are treated with the formulated test compound (0.02% active ingredient) in a spray chamber. One day after application, the grape plants are inoculated by shaking plants infected with grape powdery mildew above the test plants. After an incubation period of 7 days at 26°C and 60%r.h. under a light regime of 14/10hours (light/dark) the disease incidence is assessed.

Infestation is prevented virtually completely (0-5% infestation) with each of compounds 1.01, 1.02, 1.05, 1.06, 1.08, 1.09, 1.14, 1.15, 1.18, 1.24, 1.25, 1.28, 1.34, 1.35, 1.60, 1.61, 1.92, 1.93, 1.104, 1. 105, 2.01, 2.05, 2.24, 3.01, 3.05, 3.08, 3.24, 3.27 and 3.103.

### Example B-11: Action against Septoria tritici / wheat (Septoria leaf spot on wheat)

2 week old wheat plants cv. Riband are treated with the formulated test compound (0.2% active ingredient) in a spray chamber. One day after application, wheat plants are inoculated by spraying a spore suspension (10x10⁵conidia/ml) on the test plants. After an incubation period of 1 day at 23°C and 95% r.h., the plants are kept for 16 days at 23°C and 60% r.h. in a greenhouse. The disease incidence is assessed 18 days after inoculation.

Compounds 1.06, 1.02, 1.28, 1.27, 3.01, 1.25, 1.24, 3.24, 3.27, 2.24, 3.05, 1.34, 1.35, 1.92, 1.93, 1.08, 1.14, 1.60, 1.61, 1.105, 1.09, 3.08, 1.103 and 3.14 each show good activity in this test (<20% disease incidence).

## Claims

1. A compound of formula (I): 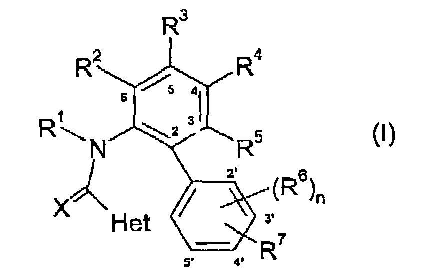 where Het is a pyrazole, pyrrole or thiazole ring, the ring being substituted by one, two or three groups R^{y};
R¹ is hydrogen;
R², R³ and R⁴ are each, independently, hydrogen, halogen, methyl or CF₃;
R⁵ is hydrogen or fluorine;
each R⁶ is, independently, halogen, methyl or CF₃;
R⁷ is (Z)ₘC≡C(Y¹);
each R^{y} is, independently, halogen, C₁₋₃ alkyl, C₁₋₃ haloalkyl, C₁₋₃ alkoxy(C₁₋₃)alkylene or cyano;
X is O;
Y¹ is hydrogen, halogen, C₁₋₄ alkyl [optionally substituted by one or more substituents each independently selected from halogen, hydroxy, C₁₋₄ alkoxy, C₁₋₄ haloalkoxy, C₁₋₄ alkylthio, C₁₋₄ haloalkylthio, C₁₋₄ alkylamino, di(C₁₋₄)alkylamino,
C₁₋₄ alkoxycarbonyl and tri(C₁₋₄)alkylsilyl], C₂₋₄ alkenyl [optionally substituted by one or more substituents each independently selected from halogen], C₂₋₄ alkynyl [optionally substituted by one or more substituents each independently selected from halogen], C₃₋₇ cycloalkyl [optionally substituted by one or more substituents each independently selected from halogen, C₁₋₄ alkyl and C₁₋₄ haloalkyl] or tri(C₁₋₄)alkylsilyl;
Z is C₁₋₄ alkylene [optionally substituted by one or more substituents each independently selected from hydroxy, cyano, C₁₋₄ alkoxy, C₁₋₄ haloalkoxy, C₁₋₄ alkylthio, COOH and COO-C₁₋₄ alkyl];
m is 0 or 1; and
n is 0, 1 or 2.

2. A compound of formula (I) as claimed in claim 1 where m = 0.

3. A compound of formula (I) as claimed in claim 1 where R⁷ is in the 4' position.

4. A compound of formula (I) as claimed in claim 1 where R⁵ is hydrogen.

5. A compound of formula (I) as claimed in claim 4 where R², R³ and R⁴ are hydrogen.

6. A compound of formula (I) as claimed in claim 1 where n = 0.

7. A compound selected from 3-difluoroniethyl-1-methyl-1H-pyrazole-4-carboxylic acid [4'-(3,3-dimethyl-but-1-ynyl)-biphenyl-2-yl]-amide and 3-difluoromethyl-1-methyl-1H-pyrazole-4-carboxylic acid (4'-but-1-ynyl-biphenyl-2-yl)-amide.

8. A compound of formula (II): 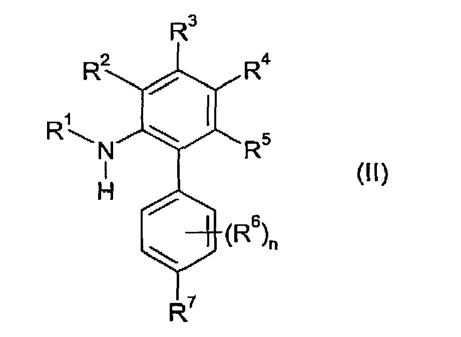 where R¹, R², R³, R⁴, R⁵, R⁶, R⁷ and n are as defined in claim 1.

9. A compound of formula (III): 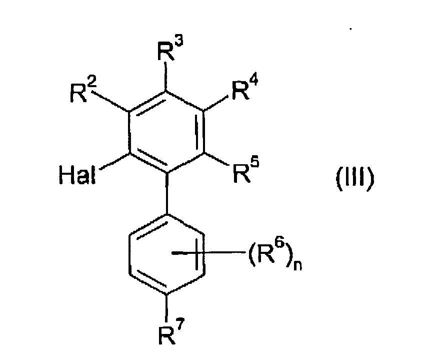 where R², R³, R⁴, R⁵, R⁶, R⁷ and n are as defined in claim 1 and Hal is bromo, chloro or iodo; provided that the compound is not a compound of formula (IIIa) 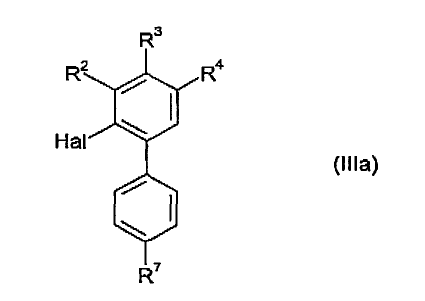 wherein Hal is Cl; R², R³ and R⁴ are each hydrogen and R⁷ is -C≡CH.

10. A composition for controlling microorganisms and preventing attack and infestation of plants therewith, wherein the active ingredient is a compound of formula (1) as claimed in claim 1 together with a suitable carrier.

11. A method of controlling or preventing infestation of cultivated plants by phytopathogenic microorganisms by application of a compound of formula (I) as claimed in claim 1 to plants, to parts thereof or the locus thereof.

## Patentansprüche

1. Verbindung der Formel (I): 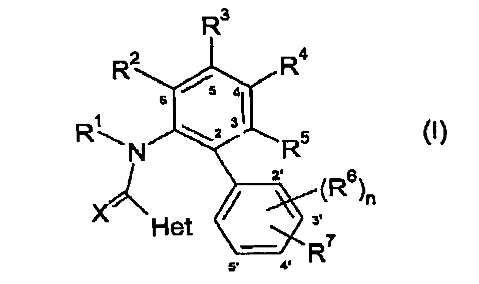 worin Het einen Pyrazol-, Pyrrol- oder Thiazolring darstellt, wobei der Ring mit einer, zwei oder drei Gruppen R^{y} substituiert ist;
R¹ Wasserstoff darstellt;
R², R³ und R⁴ jeweils unabhängig Wasserstoff, Halogen, Methyl oder CF₃ darstellen;
R⁵ Wasserstoff oder Fluor darstellt;
jedes R⁶ unabhängig Halogen, Methyl oder CF₃ darstellt;
R⁷ (Z)ₘC≡C(Y¹) darstellt;
jedes R^{y} unabhängig Halogen, C₁₋₃-Alkyl, C₁₋₃-Halogenalkyl, C₁₋₃-Alkoxy(C₁₋₃)-alkylen oder Cyano darstellt;
X O darstellt;
Y¹ Wasserstoff, Halogen, C₁₋₄-Alkyl [gegebenenfalls substituiert mit einem oder mehreren Substituenten, jeweils unabhängig ausgewählt aus Halogen, Hydroxy, C₁₋₄-Alkoxy, C₁₋₄-Halogenalkoxy, C₁₋₄-Alkylthio, C₁₋₄-Halogenalkylthio, C₁₋₄-Al-kylamino, Di(C₁₋₄)-alkylamino, C₁₋₄-Alkoxycarbonyl und Tri-(C₁₋₄)-alkylsilyl], C₂₋₄-Alkenyl [gegebenenfalls substituiert mit einem oder mehreren Substituenten, jeweils unabhängig ausgewählt aus Halogen], C₂₋₄-Alkinyl [gegebenenfalls substituiert mit einem oder mehreren Substituenten, jeweils unabhängig ausgewählt aus Halogen], C₃₋₇-Cycloalkyl [gegebenenfalls substituiert mit einem oder mehreren Substituenten, jeweils unabhängig ausgewählt aus Halogen, C₁₋₄-Alkyl und C₁₋₄-Halogenalkyl] oder Tri(C₁₋₄)-alkylsilyl darstellt;
Z C₁₋₄-Alkylen [gegebenenfalls substituiert mit einem oder mehreren Substituenten, jeweils unabhängig ausgewählt aus Hydroxy, Cyano, C₁₋₄-Alkoxy, C₁₋₄-Halogenalkoxy, C₁₋₄-Alkylthio, COOH und COO-C₁₋₄-Alkyl], darstellt;
m 0 oder 1 ist; und
n 0, 1 oder 2 ist.

2. Verbindung der Formel (I), wie in Anspruch 1 beansprucht, worin m = 0.

3. Verbindung der Formel (I), wie in Anspruch 1 beansprucht, worin R⁷ in der 4'-Position vorliegt.

4. Verbindung der Formel (I), wie in Anspruch 1 beansprucht, worin R⁵ Wasserstoff darstellt.

5. Verbindung der Formel (I), wie in Anspruch 4 beansprucht, worin R², R³ und R⁴ Wasserstoff darstellen.

6. Verbindung der Formel (I), wie in Anspruch 1 beansprucht, worin n = 0.

7. Verbindung, ausgewählt aus 3-Difluormethyl-1-methyl-1H-pyrazol-4-carbonsäure-[4'-(3,3-dimethyl-but-1-inyl)-biphenyl-2-yl]-amid und 3-Difluormethyl-1methyl-1H-pyrazol-4-carbonsäure-(4'-but-1-inyl-biphenyl-2-yl)-amid.

8. Verbindung der Formel (II): 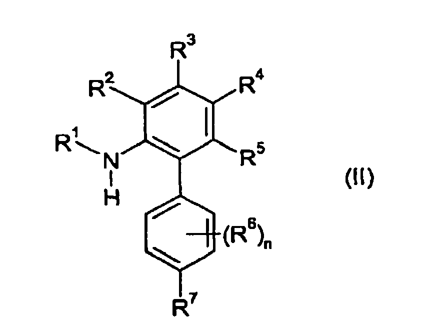 worin R¹, R², R³, R⁴, R⁵, R⁶, R⁷ und n wie in Anspruch 1 definiert sind.

9. Verbindung der Formel (III): 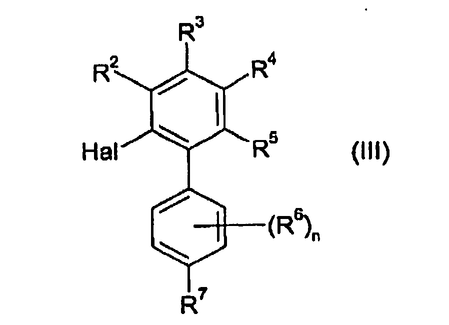 worin R², R³, R⁴, R⁵, R⁶, R⁷ und n wie in Anspruch 1 definiert sind und Hal Brom, Chlor oder Jod darstellt; mit der Maßgabe, dass die Verbindung keine Verbindung der Formel (IIIa) 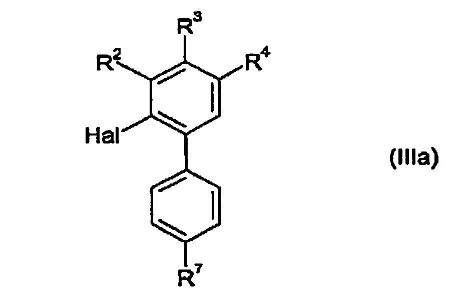 darstellt,
worin Hal Cl darstellt; R², R³ und R⁴ jeweils Wasserstoff darstellen und R⁷ -C≡CH darstellt.

10. Zusammensetzung zum Bekämpfen von Mikroorganismen und zur Prävention des Angriffs und Befalls von Pflanzen damit, wobei der Wirkbestandteil eine Verbindung der Formel (I), wie in Anspruch 1 beansprucht, zusammen mit einem geeigneten Träger, ist.

11. Verfahren zum Bekämpfen oder zur Prävention des Befalls von Kulturpflanzen durch phytopathogene Mikroorganismen durch Applikation einer Verbindung der Formel (I), wie in Anspruch 1 beansprucht, auf Pflanzen, auf Teile davon oder den Standort davon.

## Revendications

1. Composé de formule (I) : 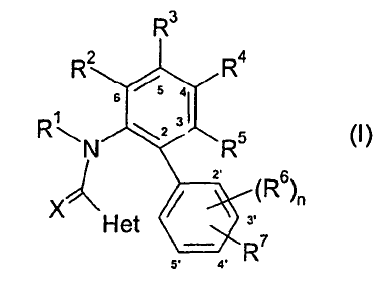 où Het est un cycle pyrazole, pyrrole ou thiazole, le cycle étant substitué par un, deux ou trois groupes R^{y} ;
R¹ est l'hydrogène ;
R², R³ et R⁴ sont chacun, indépendamment, l'hydrogène, halogène, méthyle ou CF₃ ;
R⁵ est l'hydrogène ou le fluor ;
chaque R⁶ est, indépendamment, halogène, méthyle ou CF₃ ;
R⁷ est (Z)ₘC≡C(Y¹) ;
chaque R^{y} est, indépendamment, halogène, alkyle en C₁₋₃. halogénoalkyle en C₁₋₃, (alcoxy en C₁₋₃)-(alkylène en C₁₋₃) ou cyano ;
X est O ;
Y¹ est l'hydrogène, halogène, alkyle en C₁₋₄ [éventuellement substitué par un ou plusieurs substituants choisis chacun indépendamment parmi halogène, hydroxy, alcoxy en C₁₋₄, halogénoalcoxy en C₁₋₄, alkylthio en C₁₋₄, halogénoalkylthio en C₁₋₄, (alkyl en C₁₋₄)amino, di(alkyl en C₁₋₄)amino, (alcoxy en C₁₋₄)-carbonyle et tri (alkyl en C₁₋₄)amino], alcényle en C₂₋₄ [éventuellement substitué par un ou plusieurs substituants chacun choisi indépendamment parmi halogène], alcynyle en C₂₋₄ [éventuellement substitué par un ou plusieurs substituants chacun choisi indépendamment parmi halogène], cycloalkyle en C₃₋₇ [éventuellement substitué par un ou plusieurs substituants chacun choisi indépendamment parmi halogène, alkyle en C₁₋₄ et halogénoalkyle en C₁₋₄] ou tri (alkyl en C₁₋₄)silyle ;
Z est alkylène en C₁₋₄ [éventuellement substitué par un ou plusieurs substituants chacun choisi indépendamment parmi un groupe hydroxy, cyano, alcoxy en C₁₋₄, halogénoalcoxy en C₁₋₄, alkylthio en C₁₋₄, COOH et COO-(alkyle en C₁₋₄) ;
m est 0 ou 1 ; et
n est 0, 1 ou 2.

2. Composé de formule (I) selon la revendication 1, dans laquelle m = 0.

3. Composé de formule (I) selon la revendication 1, dans laquelle R⁷ est à la 4' position.

4. Composé de formule (I) selon la revendication 1, dans laquelle R⁵ est l'hydrogène.

5. Composé de formule (I) selon la revendication 4, dans laquelle R², R³ et R⁴ sont l'hydrogène.

6. Composé de formule (I) selon la revendication 1, dans laquelle n = 0.

7. Composé choisi parmi le [4'-(3,3-diméthyl-but-1-ynyl)-biphényl-2-yl]-amide d'acide 3-difluorométhyl-1-méthyl-1H-pyrazole-4-carboxylique et le (4'-but-1-ynyl-biphényl-2-yl)-amide d'acide 3-difluorométhyl-1-méthyl-1H-pyrazole-4-carboxylique.

8. Composé de formule (II) : 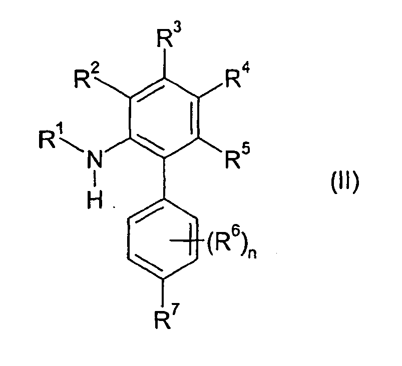 où R¹, R², R³, R⁴, R⁵, R⁶, R⁷ et n sont tels que définis dans la revendication 1.

9. Composé de formule (III) : 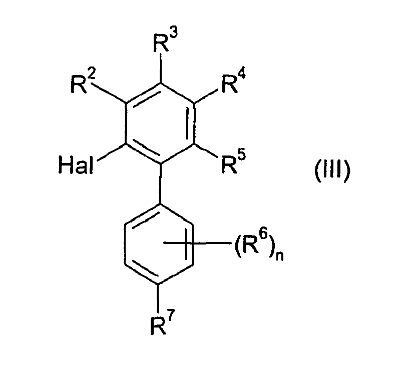 où R², R³, R⁴, R⁵, R⁶, R⁷ et n sont tels que définis dans la revendication 1, et Hal est un groupe bromo-, chloro- ou iodo- ; à condition que le composé ne soit pas un composé de formule (IIIa) : 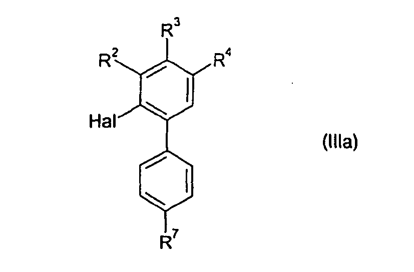 où Hal est Cl ; R², R³ et R⁴ sont chacun l'hydrogène et R⁷ est -C≡CH.

10. Composition de contrôle des micro-organismes et de prévention de l'attaque et de l'infestation des plantes par ceux-ci, dans laquelle l'ingrédient actif est un composé de formule (I) selon la revendication 1, associé à un support adapté.

11. Procédé de contrôle ou de prévention de l'infestation des plantes cultivées par des micro-organismes phytopathogènes par application d'un composé de formule (I) selon la revendication 1 sur des plantes, des parties de celles-ci ou le locus de celles-ci.
